# FASCICULE DE BREVET EUROPEEN

(11) **EP 1 315 015 B1**
(45) Date de publication et mention de la délivrance du brevet: **16.03.2005**
(21) Numéro de dépôt: 02358025.1
(22) Date de dépôt: 14.11.2002
(51) Int. Cl.: G02B 23/24, A61B 1/07

(54) **Endoscopes rotatifs à visée distale déviée**
Verdrehbare Endoskope mit abgewinkeltem Distalvisier
Rotatable endoscopes with deviated distal sight

(30) Priorité: 19.11.2001 FR 0114936
(43) Date de publication de la demande: 28.05.2003
(73) Titulaire: Tokendo (S.a.r.l.), 13600 La Ciotat (FR)
(72) Inventeur: Rovegno, Jean, 13600 La Ciotat (FR)
(74) Mandataire: de Roquemaurel, Bruno

(56) Documents cités:
- EP-A- 0 260 856
- FR-A- 2 783 937
- GB-A- 2 322 944

## Description

La présente invention concerne un endoscope à visée déviée bénéficiant de tout ou partie des facilités suivantes :
. Rotation de l'axe de visée
. Variation de l'angle de visée
. Rotation d'un réticule Réglage de mise au point

On désigne par le terme d'endoscope une sonde rigide qui, introduite dans une cavité non éclairée, permet à l'utilisateur d'observer l'intérieur de ladite cavité. Pour ce faire, un endoscope intègre par nature un dispositif optique et un dispositif d'éclairage.

On désigne par le terme d'endoscope à visée axiale un endoscope dans lequel l'axe optique de l'objectif distal est confondu avec l'axe mécanique dudit endoscope. Le dispositif optique d'un endoscope à visée axiale comprenant un hublot optique frontal, un objectif distal, un système de transport optique d'image généralement constitué d'une série de lentilles achromatiques et une lentille oculaire dont le déplacement longitudinal permet à l'utilisateur de régler la netteté de l'image observée. Ledit dispositif optique étant calculé de façon à ce que l'image transmise par la lentille oculaire ne présente pas d'inversion bi-directionnelle par rapport à la réalité. Le dispositif d'éclairage d'un endoscope à visée axiale étant constitué d'un faisceau de fibres d'éclairage dont l'extrémité distale constitue généralement une fenêtre d'éclairage en forme de couronne disposée autour du hublot optique frontal. L'extrémité proximale du faisceau de fibres d'éclairage étant logée dans une embase d'éclairage latérale intégrée dans la poignée de l'endoscope. Le champ d'éclairage crée par la fenêtre distale d'éclairage recouvrant le champ optique de l'endoscope quand l'embase d'éclairage est connectée, par l'intermédiaire d'un câble d'éclairage, à un générateur de lumière.

On désigne par le terme d'endoscope à visée déviée un endoscope dans lequel l'axe de visée optique forme un angle avec l'axe mécanique de l'endoscope. La visée étant prograde si cet angle est inférieur à 90°, latérale s'il est égal à 90° et rétrograde s'il est supérieur à 90°. Le dispositif optique d'un endoscope à visée déviée comportant donc dans tous les cas un prisme déviateur distal.

Si le prisme distal est un prisme déviateur à réflexion totale caractérisé par une inversion bi-directionnelle de l'image transmise par ledit prisme, le dispositif optique de l'endoscope comprendra un hublot optique latéral, le prisme déviateur distal, un objectif, un système optique de transport d'image généralement constitué d'une série de lentilles achromatiques et une lentille oculaire dont le déplacement longitudinal permet à l'utilisateur de régler la netteté de l'image observée. Ledit système optique de transport d'image étant calculé de façon à ce que l'image délivrée par la lentille oculaire ne soit pas totalement inversée par rapport à la réalité.

Si le prisme distal est un prisme déviateur à réflexion partielle caractérisé par une inversion unidirectionnelle de l'image transmise par ledit prisme, le dispositif optique de l'endoscope comprendra un hublot optique latéral, le prisme déviateur distal, un objectif, un système optique de transport d'image généralement constitué d'une série de lentilles achromatiques, un prisme correcteur introduisant une inversion unidirectionnelle de l'image transmise par ledit prisme et une lentille oculaire dont le déplacement longitudinal permet à l'utilisateur de régler la netteté de l'image observée. Le positionnement radial du prisme correcteur et la structure du système optique de transport d'image étant calculés de façon à ce que l'image délivrée par la lentille oculaire ne soit pas partiellement inversée par rapport à la réalité. Les endoscopes à visée déviée et mise au point réglable équipés d'un prisme déviateur distal à réflexion partielle présentent deux types principaux d'architecture. La première de ces architectures concerne les endoscopes où le prisme correcteur est directement intégré dans le système optique de transport d'image, les faibles dimensions dudit prisme correcteur constituant dans ces conditions un handicap en matière de luminosité. La seconde architecture évoquée semble-t-il pour la première fois dans un brevet datant de 1938 (Louis K-PITMAN / US PATENT 2,118,523/1938), concerne les endoscopes dont le prisme correcteur constitue l'extrémité proximale du dispositif optique. Dans ce cas, il est rapidement apparu comme très avantageux de mettre en oeuvre dans la partie proximale de l'endoscope une monture cylindrique servant de logement à la lentille oculaire, monture dont l'extrémité proximale abrite le prisme correcteur et dont l'extrémité distale contient un diaphragme de champ fixement positionné dans le plan focal distal de la lentille oculaire. Le réglage de mise au point de l'endoscope s'effectuant en déplaçant longitudinalement ladite monture. Ce type de dispositif, décrit dès 1961 par la société américaine ACMI (US PATENT 2.990.830/1961) a été depuis lors adopté par de nombreux constructeurs d'endoscopes.

Le dispositif d'éclairage d'un endoscope à visée déviée est, quant à lui, généralement constitué d'un faisceau de fibres d'éclairage dont l'extrémité distale constitue une fenêtre d'éclairage latérale située entre le hublot optique latéral et l'extrémité distale dudit endoscope, l'axe d'illumination de ladite fenêtre étant sensiblement parallèle à l'axe de visée optique dudit endoscope. L'extrémité proximale du faisceau de fibres d'éclairage étant logée dans une embase d'éclairage latérale intégrée dans la poignée de l'endoscope. Le champ d'éclairage crée par la fenêtre latérale d'éclairage recouvrant le champ optique de l'endoscope quand l'embase d'éclairage est connectée, par l'intermédiaire d'un câble d'éclairage, à un générateur de lumière.

Les difficultés d'exploitation propres aux endoscopes traditionnels à visée déviée concernent l'exploration panoramique de l'intérieur d'une cavité. Un tel examen obligera en effet l'utilisateur à faire effectuer à l'endoscope une rotation de 360° autour de son axe mécanique, opération rendue malaisée par la présence du câble d'éclairage solidaire de l'embase d'éclairage dudit endoscope. Ces problèmes d'exploitation sont à l'origine du développement d'endoscopes « rotatifs » à visée distale déviée désignés selon les constructeurs sous les termes de « Rotascope » (HENKE SASS WOLF), d' « Endoscope à coquille tournante » (EFER), de « Boroscope à connecteur de lumière rotatif » (KARL STORZ), de « Technoscope à connecteur de lumière rotatif » (RICHARD WOLF) ou de « Borescope à balayage orbital » (OLYMPUS). Tous ces endoscopes disposant d'une sonde endoscopique à visée distale déviée dont l'extrémité proximale tourne à l'intérieur d'une poignée équipée d'une bague commandant la rotation de la sonde, d'une embase latérale de connexion de câble d'éclairage, d'une bague de réglage de mise au point et d'une bonnette de vision proximale. Ce type d'architecture permettant à l'utilisateur de faire effectuer à la sonde endoscopique une rotation autour de son axe sans modifier la position du câble d'éclairage connecté à l'embase latérale d'éclairage de l'endoscope. Les dispositifs optiques mis en oeuvre dans les différents modèles d'endoscopes rotatifs cités ci-dessus pouvant être classés dans l'une des trois familles décrites ci-après.

La première famille d'endoscopes rotatifs, développée depuis de nombreuses années notamment par la société allemande HENKE-SASS WOLF, concerne des endoscopes dont le dispositif optique intégré dans la sonde endoscopique tournante est constitué d'un prisme déviateur distal à réflexion totale, d'un objectif et d'un système optique de transport d'image. L'image délivrée par l'extrémité proximale de la sonde endoscopique tournante étant transmise à une lentille oculaire fixement associée à un diaphragme de champ positionné dans son plan focal distal et logée de façon coulissante dans la poignée de l'endoscope, le déplacement longitudinal de ladite lentille oculaire étant commandé par une bague de réglage de mise au point. Le principal inconvénient du dispositif optique décrit ci-dessus résulte de l'utilisation de prismes à réflexion totale relativement coûteux et difficilement réalisables dans une large gamme de dimensions et d'angles de déviation. La société allemande HENKE-SASS WOLF a décrit en 1989 (Deutsche patent 0.371.233/1989) une poignée originale permettant d'améliorer l'ergonomie des commandes d'un endoscope rotatif de ce type.

La seconde famille d'endoscopes rotatifs, développée depuis le début des années 80 notamment par la société française EFER, concerne des endoscopes dont le dispositif optique intégré dans la sonde endoscopique tournante est constitué d'un prisme déviateur distal à réflexion partielle, d'un objectif et d'un système optique de transport d'image à l'intérieur duquel est inséré un prisme correcteur. L'image délivrée par l'extrémité proximale de la sonde endoscopique tournante étant transmise à une lentille oculaire fixement associée à un diaphragme de champ positionné dans son plan focal distal et logée de façon coulissante dans la poignée de l'endoscope, le déplacement longitudinal de ladite lentille oculaire étant commandé par une bague de réglage de mise au point. Le principal inconvénient de la solution décrite ci-dessus résulte du fait que les faibles dimensions du prisme correcteur intégré dans le système optique de transport d'image de la sonde endoscopique tournante limitent la luminosité globale de l'endoscope et interdisent en pratique la mise en oeuvre d'un tel dispositif optique dans des sondes endoscopiques de faible diamètre. Un modèle d'endoscope de ce type commercialisé à la fin des années 90 par la société japonaise OLYMPUS a néanmoins été décrit dans un brevet déposé en 1998 par la société anglaise KEYMED (UK patent 2.322.944/1998).

La troisième famille d'endoscopes rotatifs concerne les endoscopes dont le dispositif optique intégré dans la sonde endoscopique tournante est constitué d'un prisme déviateur distal à réflexion partielle, d'un objectif et d'un système optique de transport d'image. L'image délivrée par l'extrémité proximale du système optique de transport d'image intégré dans la sonde endoscopique tournante étant transmise à une lentille oculaire fixée dans une monture logée dans la poignée de l'endoscope, lentille oculaire à laquelle sont fixement associés un prisme correcteur logé dans l'extrémité proximale de ladite monture et un diaphragme de champ positionné dans son plan focal distal. L'extrémité proximale de la sonde endoscopique tournante étant mécaniquement associée à ladite monture de telle façon que le prisme déviateur distal de la sonde endoscopique et le prisme correcteur conservent le même alignement relatif lors d'une rotation de ladite sonde autour de son axe. Il convient de noter en préalable que le procédé consistant à synchroniser la rotation d'un prisme déviateur à réflexion partielle situé à l'extrémité distale d'un système optique avec celle d'un prisme correcteur situé à l'extrémité proximale dudit système optique a été abondamment décrit dans divers brevets concernant la mise en oeuvre de télescopes ou de périscopes (ERNST LEITZ GMBH / UK PATENT 1.272.742/1965, LUDWIG PIETZCH / DEUTSCHE PATENT 28 33 944/1978, THEODOR PREUSSNER / UK PATENT 2.187.303/1987, THEODOR PREUSSNER / US PATENT 4 787 725/1988). La synchronisation d'un prisme déviateur distal à réflexion partielle et d'un prisme correcteur proximal a été également mise en oeuvre dès 1977 dans un endoscope binoculaire rotatif à visée distale déviée décrit par JERRALD WIDRAN (US PATENT 4 061 135/1977). Dans le cas d'un endoscope rotatif à visée distale déviée, le dispositif de correction du sens de l'image consistant à accoupler en rotation la sonde endoscopique et la monture regroupant le diaphragme de champ, la lentille oculaire et le prisme correcteur doit en fait être associé à un dispositif de mise au point permettant de déplacer longitudinalement ladite monture par rapport à l'extrémité proximale de la sonde endoscopique.

Le procédé relevant du domaine public et consistant à associer l'extrémité proximale d'une sonde endoscopique tournante à une monture regroupant un diaphragme de champ, une lentille oculaire et un prisme correcteur proximal a été mis en oeuvre à la fin des années 80 par la société allemande RICHARD WOLF. L'architecture adoptée par ce constructeur étant caractérisée par la structure de la poignée de ces endoscopes qui présente deux parties distinctes mécaniquement accouplées en rotation : une partie distale « fixe » disposant d'une bague de commande de rotation et d'une embase latérale de connexion de câble d'éclairage, et une partie proximale « tournante » disposant d'une bague de réglage de mise au point commandant le déplacement longitudinal de la monture regroupant le diaphragme de champ, la lentille oculaire et le prisme correcteur, monture logée de façon coulissante à l'intérieur de ladite partie proximale. L'extrémité proximale de la sonde endoscopique, qui tourne librement à l'intérieur de la partie distale de la poignée, étant mécaniquement solidaire de la partie proximale de ladite poignée. La société allemande KARL STORZ a décrit en 1990(US PATENT 5.088.819/1992) une poignée originale permettant d'améliorer l'ergonomie des commandes d'un endoscope rotatif de ce type. Le principal inconvénient de la solution décrite ci-dessus résulte du fait que la rotation de la sonde endoscopique entraîne la rotation de la partie proximale de la poignée et donc la rotation de la bonnette de l'endoscope située devant l'oeil de l'utilisateur.

Une architecture originale permettant de s'affranchir de cet inconvénient majeur a été mise en oeuvre en 1992 par la société française EFER dans le cadre d'un équipement de vidéo thoracoscopie constitué d'une sonde endoscopique rotative à visée latérale disposant d'une commande de mise au point proximale associée à une caméra vidéo. Une architecture similaire à été ensuite mise en oeuvre dans les endoscopes rotatifs à visée distale déviée commercialisés par la société japonaise OLYMPUS et décrits dans les brevets déposés en 1993 par la société anglaise KEYMED (UK PATENT GB 2 280 514, EUROPEAN PATENT EP 0 636 915, US PATENT 5 540 650). Une architecture comparable a été également décrite dans un brevet déposé en 1998 par la société française TOKENDO (brevet français FR 97 04569). Tous les endoscopes relevant de ce type d'architecture sont caractérisés par des dispositifs spécifiques d'accouplement mécanique dont bénéficie la monture regroupant le diaphragme de champ, la lentille oculaire et le prisme correcteur, monture logée de façon coulissante et tournante à l'intérieur de la poignée desdits endoscopes. Un premier dispositif d'accouplement de nature coulissante permettant à l'extrémité proximale de la sonde endoscopique de transmettre son mouvement de rotation à ladite monture, et ce quel que soit le positionnement longitudinal de ladite monture dans la poignée. Un second dispositif d'accouplement permettant à la bague de réglage de mise au point de déplacer longitudinalement dans la poignée ladite monture, et ce quel que soit le positionnement radial de ladite monture dans la poignée. La solution décrite ci-dessus, bien que notablement améliorée en 2000 par la société française TOKENDO (brevet français FR 98 12404) n'en présente pas moins de sérieux inconvénients résultant des tolérances mécaniques que présentent nécessairement les dispositifs cinématiques complexes associés à la monture regroupant le diaphragme de champ, la lentille oculaire et le prisme correcteur, tolérances entraînant :
. Des variations d'alignement angulaire entre le prisme déviateur distal de la sonde endoscopique et le prisme correcteur, variations se traduisant par des défauts d'orientation aléatoires de l'image délivrée par l'endoscope.
. Des variations de centrage du prisme correcteur sur l'axe optique de l'endoscope, variations se traduisant par des déviations angulaires aléatoires de l'axe de sortie de l'image délivrée par l'endoscope.

L'endoscope rotatif à visée distale déviée et mise au point proximale décrit en 2000 par la société française TOKENDO (brevet français FR 98 11826 / brevet allemand DE 19942 152 A1 / Brevet américain 6.346.076 / Brevet britannique 2.342.462) bénéficie d'une structure opto-mécanique originale permettant de s'affranchir définitivement des défauts mécaniques d'alignement et de centrage évoqués ci-dessus. Le prisme correcteur de cet endoscope est en effet fixé à demeure dans l'extrémité proximale d'un tube cylindrique logé de façon tournante dans la poignée dudit endoscope et dont l'extrémité distale est fixement solidaire de l'extrémité proximale de la sonde endoscopique rigide tournante associée à ladite poignée. La lentille oculaire dudit endoscope et le diaphragme de champ qui lui est associé étant fixés dans une monture cylindrique logée de façon coulissante dans la partie médiane du tube cylindrique. Un dispositif d'accouplement mécanique associé à une bague externe de commande de mise au point permet de déplacer longitudinalement ladite monture à l'intérieur du tube cylindrique. Ledit dispositif d'accouplement évitant toute interférence entre le mouvement de rotation du tube cylindrique et le mouvement de translation longitudinale à l'intérieur dudit tube de la monture de la lentille oculaire.

Les différents types d'endoscopes rigides rotatifs à visée distale déviée décrits ci-dessus facilitent donc l'exploration panoramique de l'intérieur d'une cavité en permettant à l'utilisateur de faire tourner sur 360° l'axe de visée desdits endoscopes. Une autre catégorie d'endoscopes rigides répond au même souci ergonomique : il s'agit d'endoscopes, généralement nommés « endoscopes à prisme basculant » dont l'utilisateur peut faire varier l'angle de visée optique. Les dispositifs opto-mécaniques mis en oeuvre dans un tel contexte peuvent relever de deux concepts différents.

Le premier de ces concepts, décrit en 1987 de façon détaillée par la société américaine BAXTER (US PATENT 4 697 577) concerne des endoscopes dont la rotation de l'axe de visée dans un plan parallèle à l'axe de la sonde endoscopique met en oeuvre un dispositif de déviation distal constitué de deux prismes déviateurs à réflexion partielle. Le premier de ces prismes, fixement associé à l'extrémité distale de l'objectif de l'endoscope, introduit une déviation optique de 90°. Ce prisme fixe est associé à un second prisme mobile introduisant également une déviation de 90° et susceptible de tourner autour d'un axe perpendiculaire à l'axe optique de l'objectif de l'endoscope de façon à ce que l' axe optique d'entrée du prisme fixe et l'axe optique de sortie du prisme mobile restent confondus lors de la rotation dudit prisme mobile. Un tel endoscope nécessite un dispositif de correction proximal constitué de deux prismes correcteurs à réflexion partielle logés dans la poignée dudit endoscope. Le premier de ces prismes est un prisme fixe destiné à corriger la réflexion unidirectionnelle introduite par le prisme fixe du dispositif de déviation distal. Ce prisme fixe est associé à un second prisme mobile tournant en synchronisme avec le prisme mobile du dispositif de déviation distal de façon à corriger en permanence la réflexion unidirectionnelle introduite par ledit prisme. Une architecture distale similaire à celle qui vient d'être évoquée a été décrite en 1999 par la société anglaise KEYMED (WO 0122865/2001). Tous les endoscopes relevant du concept évoqué ci-dessus bénéficient d'une course de variation d'angle de visée très importante (de l'ordre de 120°) permettant d'effectuer des observations axiale, prograde, latérale et rétrograde. La fragilité intrinsèque du hublot optique distal spécifique à ce type d'endoscope rend par contre très marginale leur utilisation en milieu industriel.

Le second concept d'endoscope à prisme basculant concerne des endoscopes à visée déviée dont la rotation de l'axe de visée dans un plan contenant l'axe optique de la sonde endoscopique met en oeuvre un unique prisme déviateur à réflexion partielle logé devant l'extrémité distale de l'objectif de l'endoscope et susceptible de tourner autour d'un axe perpendiculaire à l'axe optique de l'objectif de façon à ce que l'axe optique de sortie dudit prisme coïncide avec l'axe optique dudit objectif. Le dispositif optique permettant de corriger la réflexion partielle introduite par le prisme déviateur distal peut être dans ce cas constitué d'un simple prisme correcteur à réflexion partielle directement intégré dans le système optique de transport d'image de l'endoscope suivant un procédé décrit en 1965 par le Commissariat Français à l'Energie Atomique (UK PATENT 1.155.390/1966), procédé dont les inconvénients en matière de luminosité ont déjà été évoqués ci-dessus. Le dispositif proximal de correction et de mise au point intégré dans la poignée d'un bronchoscope conçu en Russie et ayant fait l'objet en 1963 d'un dépôt de brevet aux USA (US PATENT 3.096.756) constitue une solution plus avantageuse. Ce bronchoscope met en oeuvre une monture cylindrique servant de logement à la lentille oculaire, dont l'extrémité proximale abrite le prisme correcteur, et dont l'extrémité distale contient un diaphragme de champ fixement positionné dans le plan focal distal de la lentille oculaire. Le réglage de mise au point dudit bronchoscope s'effectuant en déplaçant longitudinalement ladite monture. La course de variation d'angle de visée de ce type d'endoscopes, pratiquement limitée à environ 70°, ne permet pas d'effectuer des observations axiales. Leur architecture distale permet par contre de réaliser des sondes endoscopiques de faible diamètre particulièrement robustes.

Il est apparu rapidement très intéressant d'améliorer les caractéristiques d'un endoscope rigide à visée déviée en intégrant simultanément dans la poignée dudit endoscope un dispositif de mise au point proximale, un dispositif de commande de rotation de l'axe de visée et un dispositif de commande de basculement du prisme déviateur distal. La société allemande KARL STORZ a décrit en 1998 (WO 00/1199) une poignée d'endoscope rigide à visée déviée intégrant les trois dispositifs évoqués ci-dessus. Mais si ce document décrit de façon détaillée l'ergonomie de ladite poignée, il ne fournit par contre aucune information sur les moyens optiques et mécaniques permettant de parvenir à un tel résultat. Le seul dispositif significatif décrit dans ledit document concerne le basculement du prisme déviateur distal qui est commandé par le déplacement longitudinal d'une tige métallique disposée parallèlement au tube servant de logement au système optique de transport d'image, architecture dont l'encombrement s'avère peu propice à la réalisation d'endoscopes de faible diamètre.

Une façon simple d'estimer les dimensions des défauts de surface d'une pièce mécanique observée à l'aide d'un endoscope consiste à positionner un réticule dans le plan focal distal de la lentille oculaire dudit endoscope. Dans de telles conditions, l'image dudit réticule se trouve superposée à l'image endoscopique observée par l'utilisateur, et ce même si un dispositif de mise au point proximale permet de déplacer longitudinalement une monture intégrant la lentille oculaire et ledit réticule. Il apparaît alors intéressant d'offrir à l'utilisateur un moyen simple de « faire tourner le réticule dans l'image », ledit moyen consistant simplement à faire tourner autour de son axe la monture cylindrique dans laquelle sont logés le réticule et la lentille oculaire. Le document WO 99 56165 déposé en 1998 par la société anglaise KEYMED décrit un endoscope rigide à visée déviée intégrant simultanément un dispositif de rotation de l'axe de visée, un dispositif de mise au point proximale, et un dispositif de rotation du réticule associé à la lentille oculaire dudit endoscope. Le domaine d'application de l'endoscope décrit dans ledit document s'avère sévèrement limité en raison de l'intégration du prisme correcteur dans le système optique de transport d'image, disposition dont les inconvénients en matière de luminosité ont été ci-dessus soulignés à plusieurs reprises.

La présente invention s'est donnée pour but de décrire un endoscope à visée distale déviée équipé d'un prisme distal à réflexion partielle et d'un prisme correcteur proximal de grandes dimensions, et susceptible d'intégrer tout ou partie des dispositifs suivants :
. Dispositif de rotation de l'axe de visée
. Dispositif de basculement de l'axe de visée
. Dispositif de mise au point proximale
. Dispositif de rotation de réticule

### DESCRIPTION SOMMAIRE DE L'INVENTION

La présente invention concerne des endoscopes à sonde tournante et à visée distale déviée dont la structure résulte de l'association des dispositifs décrits ci-après.
I/ Un ensemble mécanique regroupant : une poignée tubulaire cylindrique, et une sonde comprenant un tube cylindrique interne fixement associé à un tube cylindrique externe dont l'extrémité proximale est fixement solidaire d'une bague de commande de rotation de l'axe de visée, entraînant la sonde en rotation axiale et ceinturant de façon tournante l'extrémité distale de la poignée, ladite bague étant fixement solidaire d'un tube cylindrique tournant logé de façon tournante dans la poignée, le prolongement proximal du tube interne débouchant dans le tube tournant.
II/ Un dispositif optique comportant un hublot latéral intégré dans la partie distale du tube externe, un prisme déviateur et un objectif logés dans la partie distale du tube interne, un système optique de transport d'image logé dans le tube interne, un oculaire disposé dans le tube tournant et un hublot d'observation fermant l'orifice proximal de la poignée.

Selon l'invention, la poignée comprend plusieurs dispositifs de commande totalement indépendants, chacun desdits dispositifs comprenant une bague de commande ceinturant de façon tournante la poignée, une monture cylindrique logée de façon coulissante dans le tube tournant, et un système d'accouplement associant la bague de commande à la monture cylindrique, de manière à ce qu'un mouvement de rotation de la bague de commande entraîne un mouvement de translation longitudinal de la monture à l'intérieur du tube tournant, le mouvement de rotation du tube tournant et les mouvements de rotation de la bague de commande et de translation de la monture de chacun desdits dispositifs de commande étant indépendants les uns des autres.

Les dispositifs évoqués ci-dessus permettent de réaliser et d'intégrer dans un endoscope rotatif à visée distale déviée tout ou partie des fonctions décrites ci-après à titre d'exemple.
. Commande de mise au point: dispositif permettant de déplacer longitudinalement une monture cylindrique servant de logement à la lentille oculaire de l'endoscope.
. Commande de variation de visée : dispositif permettant de déplacer longitudinalement une monture cylindrique constituant l'extrémité proximale d'un tube de manoeuvre logé de façon coulissante autour du tube interne de la sonde. Le déplacement longitudinal de l'extrémité distale dudit tube de manoeuvre commandant le basculement autour d'un axe perpendiculaire à la sonde du prisme déviateur distal à réflexion partielle de l'endoscope.
. Commande de rotation de réticule: dispositif permettant de déplacer longitudinalement une monture cylindrique disposant d'un doigt cylindrique radial logé de façon circulante dans une fente hélicoïdale ménagée dans la partie distale d'un tube cylindrique dont l'extrémité proximale sert de logement à un réticule transparent positionné dans le plan focal distal de la lentille oculaire de l'endoscope.

### PRESENTATION SOMMAIRE DES FIGURES ILLUSTRANT L'INVENTION

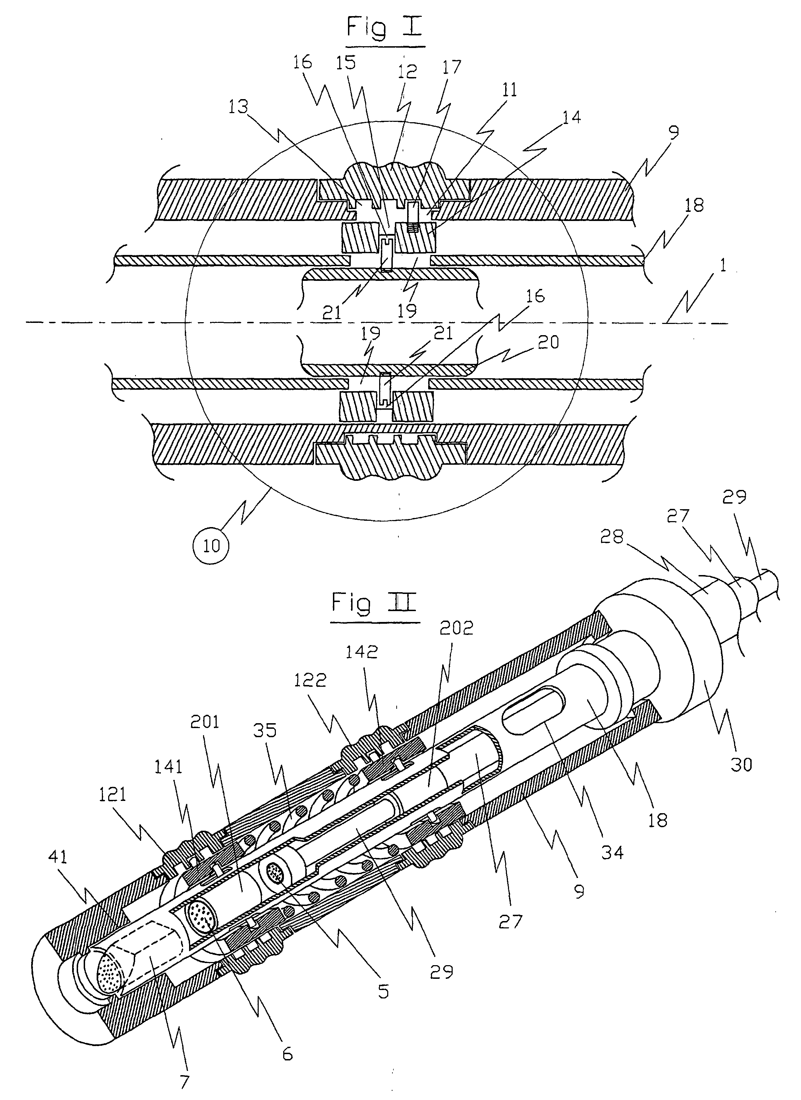

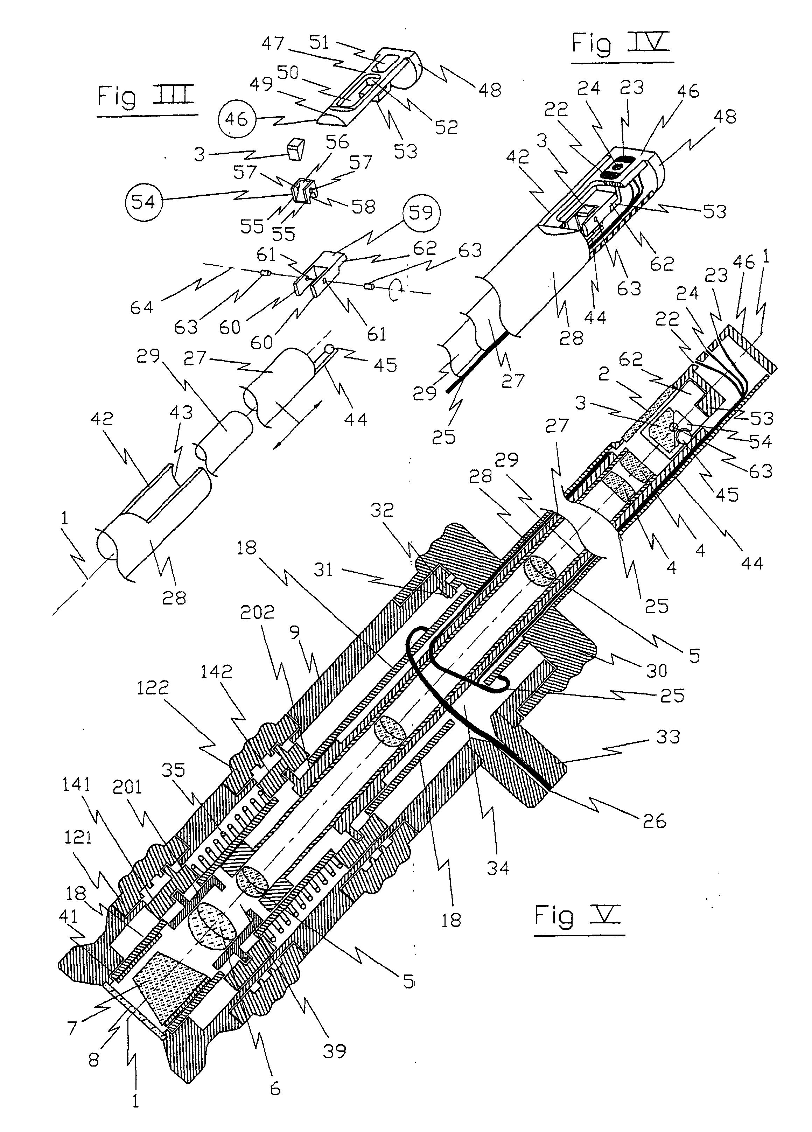

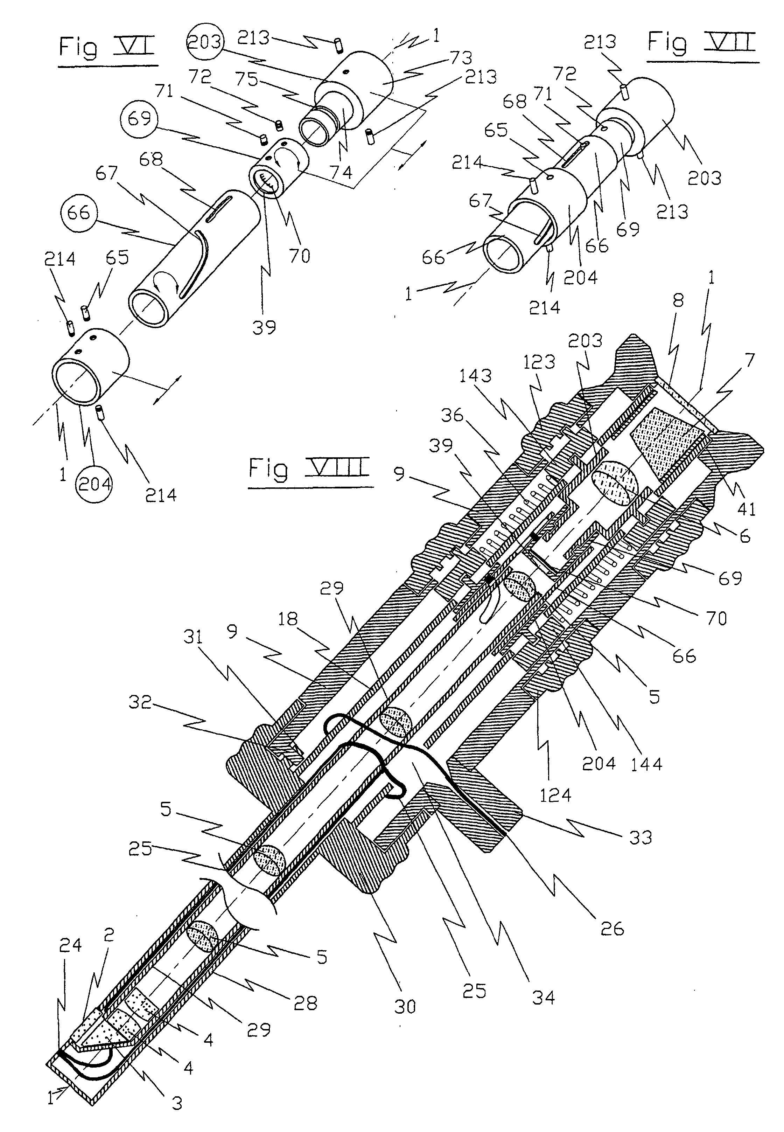

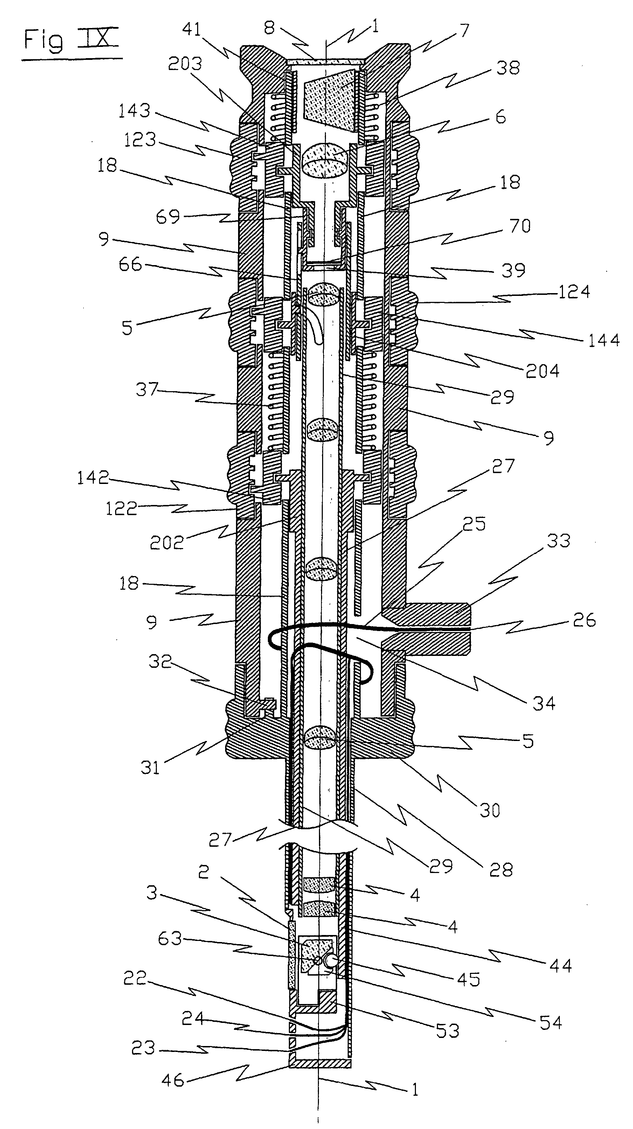

La figure I illustre la structure des dispositifs de commande mis en oeuvre dans les endoscopes rotatifs à visée distale déviée relevant de la présente invention.
La figure II illustre la structure de la poignée d'un endoscope rotatif équipé d'un prisme déviateur distal à réflexion partielle, relevant de la présente invention et bénéficiant simultanément d'une commande de mise au point et d'une commande de variation d'angle de visée.
La figure III illustre les divers composants mécaniques spécifiques intégrés dans l'extrémité distale d'un endoscope à visée distale déviée bénéficiant d'un dispositif de variation d'angle de visée relevant de la présente invention.
La figure IV illustre la structure distale d'un endoscope à visée distale déviée bénéficiant d'un dispositif de variation d'angle de visée relevant de la présente invention.
La figure V illustre la structure d'un endoscope rotatif équipé d'un prisme déviateur distal à réflexion partielle, relevant de la présente invention, et bénéficiant simultanément d'une commande de mise au point et d'une commande de variation d'angle de visée.
La figure VI illustre les divers composants mécaniques constituant un dispositif d'accouplement spécifique intégré dans la poignée d'un endoscope rotatif à visée distale déviée relevant de la présente invention et bénéficiant simultanément d'une commande de mise au point et d'une commande de rotation de réticule.
La figure VII illustre le dispositif d'accouplement spécifique intégré dans la poignée d'un endoscope rotatif à visée distale déviée relevant de la présente invention et bénéficiant simultanément d'une commande de mise au point et d'une commande de rotation de réticule.
La figure VIII illustre la structure d'un endoscope rotatif équipé d'un prisme déviateur distal à réflexion partielle, relevant de la présente invention, et bénéficiant simultanément d'une commande de mise au point et d'une commande de rotation de réticule.
La figure IX illustre la structure d'un endoscope rotatif équipé d'un prisme déviateur distal à réflexion partielle, relevant de la présente invention, et bénéficiant simultanément d'une commande de mise au point, d'une commande de rotation de réticule et d'une commande de variation d'angle de visée.

### DESCRIPTION DETAILLEE DE L'INVENTION

### FIGURE I

La figure I illustre les principes de fonctionnement d'un dispositif cinématique 10 permettant de commander l'une quelconque des diverses fonctions (mise au point, variation de l'angle de visée, rotation d'un réticule...) susceptibles d'être intégrées dans les endoscopes rotatifs à visée distale déviée faisant l'objet de la présente invention. Un tel dispositif 10 est commandé par une bague externe 12 ceinturant de façon tournante la poignée cylindrique 9 de l'endoscope, la rotation de ladite bague entraînant le déplacement longitudinal d'une monture cylindrique 20 logée de façon coulissante à l'intérieur d'un tube cylindrique 18 solidaire de la sonde endoscopique tournante associée à la poignée cylindrique 9 et logé de façon tournante dans ladite poignée.

La rotation autour de son axe 1 dé la sonde endoscopique à visée distale déviée entraîne la rotation à l'intérieur de la poignée cylindrique 9 du tube cylindrique 18 fixement solidaire de l'extrémité proximale de ladite sonde endoscopique.

Une monture cylindrique 20, logée de façon coulissante dans le tube 18, dispose de deux doigts cylindriques radiaux externes 21 diamétralement opposés et circulant dans deux fentes longitudinales 19 ménagées de façon diamétralement opposées dans le tube 18. Les extrémités des doigts 21 étant logées de façon circulante dans une gorge annulaire interne à profil de préférence carré 16 ménagée dans une bague 14 ceinturant le tube 18. Ladite bague disposant de deux orifices radiaux 15 diamétralement opposés et débouchant dans la gorge annulaire interne 16, lesdits orifices permettant de visser dans la monture cylindrique 20 les deux doigts cylindriques 21 après que la bague 14 ait été positionnée autour du tube 18.

La bague 14 dispose d'un doigt cylindrique radial externe 17 logé de façon coulissante dans une fente longitudinale 11 ménagée dans la poignée cylindrique 9 de l'endoscope, l'extrémité dudit doigt cylindrique étant logée de façon circulante dans une gorge hélicoïdale interne à profil de préférence carré 13 ménagée dans la bague de commande 12 ceinturant la poignée cylindrique 9.

Le dispositif 10 ainsi réalisé permet de déplacer longitudinalement la monture 20 à l'intérieur du tube 18, et ce quel que soit le positionnement radial dudit tube. Ledit dispositif permet également au tube cylindrique 18, et donc à la monture 20 qui lui est associée, de tourner librement à l'intérieur de la poignée 9, et ce quel que soit le positionnement longitudinal de ladite monture.

### FIGURE II

La figure illustre les principes de fonctionnement des dispositifs 10 mis en oeuvre dans la poignée 9 d'un endoscope à visée déviée relevant de la présente invention et bénéficiant simultanément des facilités suivantes :
. Rotation de l'axe de visée autour de l'axe de l'endoscope.
. Variation de l'angle de visée de l'endoscope.
. Réglage de mise au point.

La sonde endoscopique à visée distale déviée solidaire de la poignée de commande 9 est mécaniquement constituée d'un tube interne 29 dans lequel est logé le système optique de transport d'image de l'endoscope, d'un tube de manoeuvre 27 coulissant longitudinalement autour du tube 29 et servant de transmission au dispositif de commande de variation de l'angle de visée, et d'un tube externe 28 fixement solidaire du tube interne 29.

L'extrémité proximale du tube externe 28 de la sonde endoscopique est fixement solidaire d'une bague de commande rotation 30 associée de façon tournante à l'extrémité distale de la poignée de commande 9. La rotation autour de son axe de la sonde endoscopique à visée distale déviée logée dans le tube externe 28 entraîne la rotation à l'intérieur de la poignée cylindrique 9 du tube cylindrique 18 dont l'extrémité distale est fixement solidaire de la bague de commande rotation 30 et dont l'extrémité proximale sert de logement à un prisme optique correcteur 7. La partie distale du tube 18 dispose d'un orifice 34 servant de passage à la partie proximale d'un faisceau de fibres d'éclairage logé dans le volume annulaire compris entre les tubes 27 et 28 de la sonde endoscopique.

Un premier dispositif de commande analogue au dispositif 10 décrit ci-dessus dans le texte relatif à la figure I est intégré dans la partie proximale de la poignée de commande 9. La rotation de la bague de commande externe 121 dudit dispositif entraînant le déplacement longitudinal d'une bague intermédiaire 141, déplacement qui entraîne lui-même le déplacement longitudinal à l'intérieur du tube 18 d'une monture cylindrique 201 dans laquelle est fixement logée une lentille oculaire 6. Ladite lentille oculaire restant positionnée durant toute sa course entre la lentille achromatique 5 constituant l'extrémité proximale du système optique de transport d'image logé dans le tube 29 et le prisme correcteur 7 fixement logé dans l'extrémité proximale du tube 18. Ledit dispositif permettant dans ces conditions à l'utilisateur de régler la netteté de l'image transmise par le prisme correcteur 7.

Un second dispositif de commande également analogue au dispositif 10 décrit ci-dessus dans le texte relatif à la figure I est intégré dans la partie centrale de la poignée de commande 9. La rotation de la bague de commande externe 122 dudit dispositif entraînant le déplacement longitudinal d'une bague intermédiaire 142, déplacement qui entraîne lui-même le déplacement longitudinal à l'intérieur du tube 18 d'une monture cylindrique 202 ceinturant fixement l'extrémité proximale du tube 27 coulissant autour et le long du tube 29 dans lequel est logé le système optique de transport optique d'image de l'endoscope. Le déplacement longitudinal de l'extrémité distale du tube 27 commandant lui-même le basculement du prisme déviateur distal de l'endoscope suivant des modalités qui seront ci-après explicitées dans les textes relatifs aux figures III, IV et V. Ledit dispositif permettant dans ces conditions à l'utilisateur de régler l'angle de visée de l'endoscope à visée déviée dans lequel il est intégré.

Un ressort hélicoïdal 35 ceinturant le tube 18 est légèrement comprimé entre la face proximale de la bague 142 et la face distale de la bague 141, ledit ressort étant destiné à rattraper les jeux de fonctionnement des deux dispositifs de commande évoqués ci-dessus.

### FIGURE III

La figure III illustre les divers composants spécifiques intégrés dans l'extrémité distale d'un endoscope à visée déviée relevant de la présente invention et bénéficiant d'un dispositif de commande de variation de l'angle de visée.

Le composant mécanique 46 est un embout distal destiné à être associé à l'extrémité distale du tube externe 28 de la sonde endoscopique. Ledit embout présentant une face transversale distale destinée à venir en appui sur la face distale 43 du tube 28 et une face longitudinale plate 47 se terminant par une extrémité proximale de raccordement 49, ladite face longitudinale étant destinée à venir en appui sur l'échancrure 42 ménagée dans l'extrémité distale du tube 28. La face longitudinale plate 47 de l'embout 46 dispose d'un orifice distal 51 destiné à recevoir l'extrémité distale du faisceau de fibres d'éclairage de l'endoscope et d'un orifice proximal 50 destiné à recevoir un hublot de verre servant de protection à l'extrémité distale du dispositif optique de l'endoscope. L'embout 46 dispose d'une protubérance interne 53 présentant la forme d'une équerre dont la partie longitudinale est destinée à servir de support à l'extrémité distale de l'étrier 59.

Le composant mécanique 54 est destiné à servir de support au prisme déviateur 3 constituant l'extrémité distale du dispositif optique de l'endoscope. Ce composant dispose de deux oreilles latérales 55 entre lesquelles est logé le prisme 3 et d'une face interne inclinée 56 destinée à servir de support à la face réfléchissante dudit prisme. Les oreilles 55 disposent chacune d'un orifice transversal 57 destiné à servir de réceptacle aux extrémités de deux doigts cylindriques 63 servant d'axe de pivotement . La face inférieure du support de prisme 54 présente par ailleurs une gorge semi-cylindrique transversale 58 destinée à servir de logement à la sphère métallique 45 fixement solidaire de la languette 44 constituant l'extrémité distale du tube de commande 27.

Le composant mécanique 59 est un étrier à l'intérieur duquel peut pivoter le support 54 du prisme déviateur distal 3. Cet étrier 59 dispose d'une extrémité distale 62 destinée à être fixement associée à la partie longitudinale de l'équerre 53 de l'embout distal 46, et de deux oreilles proximales 60 entre lesquelles peut pivoter le support de prisme 54. Les oreilles 60 disposant chacune d'un orifice transversal 61 destinés à servir de logement à deux doigts cylindriques 63 matérialisant l'axe transversal de pivotement 64.

La sonde endoscopique à visée distale déviée est mécaniquement constituée d'un tube interne 29 dans lequel est logé le système optique de transport d'image de l'endoscope, d'un tube externe 28 fixement associé au tube interne 29, et d'un tube de manoeuvre 27 coulissant longitudinalement autour et le long du tube interne 29 et dont le déplacement longitudinal sert à commander le basculement du prisme déviateur distal 3. Le tube externe 28 présentant une échancrure 42 destinée à servir de support à l'embout distal 46, et le tube de commande 27 disposant d'une languette distale 44 sur laquelle est fixée la sphère métallique 45 dont la rotation dans la gorge 58 ménagée dans le support de prisme 54 sert à commander le basculement dudit prisme.

### FIGURE IV

La figure IV illustre la structure de l'extrémité distale d'un endoscope à visée déviée relevant de la présente invention et bénéficiant d'un dispositif de commande de variation de l'angle de visée.

L'embout distal 46 est fixement solidaire de l'extrémité distale du tube externe 28, et l'extrémité distale 62 de l'étrier servant de logement au support du prisme déviateur 3 est fixement solidaire de la branche longitudinale 53 de l'équerre interne dont dispose l'embout distal 46. Le déplacement longitudinal de l'extrémité distale du tube de manoeuvre 27 entraîne le basculement du prisme déviateur autour d'un axe transversal 63.

La portion distale du faisceau de fibres d'éclairage 25 logé dans l'espace annulaire compris entre le tube externe 28 et le tube de manoeuvre 27 est divisée en trois sous-faisceaux dont les extrémités distales sont introduites puis collées dans trois fenêtres d'éclairage 22/23/24 ménagées dans l'orifice latéral distal de l'embout 46. Le prisme 3 est quant à lui protégé par un hublot optique collé dans l'orifice latéral proximal de l'embout 46.

### FIGURE V

La figure V illustre la structure d'un endoscope à visée déviée relevant de la présente invention et bénéficiant simultanément des dispositifs suivants :
. Rotation de l'axe de visée autour de l'axe 1 de l'endoscope
. Variation de l'angle de visée de l'endoscope
. Réglage de mise au point

La structure mécanique de la sonde endoscopique est organisée autour d'un tube métallique externe 28 dont l'extrémité distale est fixement solidaire d'un embout distal 46, d'un tube interne 29 fixement solidaire du tube externe 28, et d'un tube de manoeuvre 27 ceinturant de façon coulissante le tube interne 29. L'extrémité proximale du tube externe 28 de la sonde endoscopique est fixement solidaire d'une bague de commande de rotation 30 ceinturant de façon tournante l'extrémité distale de la poignée cylindrique 9 de l'endoscope. Un dispositif de butée limitant la plage de rotation de la sonde endoscopique est constitué d'un doigt longitudinal 31 solidaire de la face proximale de la bague 30 et disposé de façon à venir en contact avec un doigt radial interne 32 solidaire de la poignée 9. La face proximale de la bague de commande de rotation 30 est fixement solidaire de l'extrémité distale d'un tube cylindrique 18 logé de façon tournante dans la poignée cylindrique 9 de l'endoscope et dont l'extrémité proximale tourne librement dans un palier 41 ménagé à cet effet à l'intérieur de l'extrémité proximale de ladite poignée.

Le dispositif optique de l'endoscope est constitué d'un hublot latéral 2 intégré dans l'embout distal 46, d'un prisme déviateur à réflexion partielle 3 également intégré dans l'embout distal 46, d'un objectif constitué de plusieurs lentilles 4 logées dans l'extrémité distale du tube interne 29, d'un système optique de transport d'image constitué d'une série de lentilles achromatiques 5 disposées dans le tube interne 29, d'une lentille oculaire 6 logée de façon coulissante dans le tube cylindrique 18, d'un prisme correcteur 7 fixement logé dans l'extrémité proximale du tube cylindrique 18 et radialement positionné de façon à compenser l'inversion unidirectionnelle d'image introduite par le prisme déviateur distal 3, et d'une glace de protection 8 fixement logée dans l'extrémité proximale de la poignée cylindrique 9.

Le dispositif d'illumination intégré dans l'endoscope comprend un faisceau 25 de fibres optiques d'éclairage logé dans le volume annulaire compris entre le tube coulissant 27 et le tube externe 28. L'extrémité distale du faisceau de fibres 25 est divisée en trois sous-faisceaux dont les extrémités distales sont collées puis polies dans trois fenêtres d'illumination latérale 22/23/24 ménagées dans l'embout distal 46. Les directions desdites fenêtres étant calculées de telle façon que la fenêtre 24 corresponde à une illumination latérale, la fenêtre 22 à une illumination rétrograde, et la fenêtre 23 à une illumination prograde. La partie proximale du faisceau de fibres 25 débouche dans le volume annulaire compris entre le tube 18 et la partie distale de la poignée cylindrique 9 à travers une fenêtre 34 ménagée dans la partie distale du tube cylindrique 18. L'extrémité proximale du faisceau de fibres 25 est collée puis polie dans l'embase latérale 33 fixement solidaire de la partie distale de la poignée 9. Plusieurs spires du faisceau de fibres 25 sont enroulées autour du tube cylindrique 18 dans le volume annulaire compris entre ledit tube et la poignée cylindrique afin d'éviter qu'une rotation de 360° de la sonde endoscopique n'entraîne des contraintes mécaniques dangereuses sur ledit faisceau de fibres.

Un premier dispositif de commande analogue au dispositif 10 précédemment décrit dans le texte relatif à la figure I est intégré dans la partie proximale de la poignée de commande 9. La rotation de la bague de commande externe 121 dudit dispositif entraînant le déplacement longitudinal de la bague 141 ceinturant le tube 18, déplacement qui entraîne lui-même le déplacement longitudinal à l'intérieur du tube cylindrique 18 de la monture cylindrique 201 dans laquelle est fixement logée la lentille oculaire 6 et dont l'orifice distal 39, positionné dans le plan focal distal de ladite lentille oculaire, fait office de diaphragme de champ.

Un second dispositif de commande également analogue au dispositif 10 précédemment décrit dans le texte relatif à la figure I est intégré dans la partie centrale de la poignée de commande 9. La rotation de la bague de commande externe 122 dudit dispositif entraînant le déplacement longitudinal de la bague 142, déplacement qui entraîne lui-même le déplacement longitudinal à l'intérieur du tube 18 de la monture cylindrique 202 ceinturant fixement l'extrémité proximale du tube de commande 27 coulissant autour du tube interne 29. Le déplacement longitudinal de la bille sphérique 45 fixement solidaire de la languette longitudinale 44 constituant l'extrémité distale du tube 27 entraînant le basculement autour de l'axe transversal 63 du support mécanique 54 du prisme déviateur 3. Ledit support 54 étant logé dans un étrier dont l'extrémité distale 62 est fixement solidaire de la branche longitudinale 53 du support en forme d'équerre disposé à l'intérieur de l'embout distal 46.

### FIGURE VI

La figure VI illustre les divers composants spécifiques intégrés dans la poignée 9 d'un endoscope à visée déviée relevant de la présente invention et bénéficiant simultanément des dispositifs suivants :
. Rotation de l'axe de visée autour de l'axe 1 de l'endoscope
. Rotation d'un réticule
. Réglage de mise au point

Le composant mécanique 203 est une pièce destinée à être logée de façon coulissante dans la partie proximale du tube 18 solidaire de la sonde endoscopique et logé de façon tournante dans la poignée de commande 9 de ladite sonde. Le composant tubulaire 203 dispose d'une partie cylindrique proximale 73 servant de monture à la lentille oculaire de l'endoscope et une partie cylindrique distale 74 de plus faible diamètre présentant une gorge annulaire externe 75.

Le composant mécanique 69 est un tube cylindrique dont la partie proximale est destinée à ceinturer la partie cylindrique distale 74 du composant 203 et dont l'orifice distal 39, faisant office de diaphragme de champ, sert de logement à un support transparent 70 sur lequel est gravé le réticule de l'endoscope. Un doigt transversal fileté 72 vissable dans la partie proximale du composant 69, et dont l'extrémité interne est destinée à venir se loger dans la gorge annulaire externe 75 usinée autour de l'extrémité distale 74 du composant 203, permet d'associer de façon tournante les composants 69 et 203. L'association desdites pièces s'effectuant de façon à ce que le réticule 70 logé dans l'orifice distal 39 du composant 69 se trouve positionné dans le plan focal distal de la lentille oculaire fixement logée dans la partie proximale 73 du composant mécanique 203.

Le composant mécanique 66 est un tube cylindrique dont la partie proximale disposant d'une fente longitudinale 68 est destinée à ceinturer la partie distale du composant mécanique 69, et dont la partie distale disposant d'une fente hélicoïdale 67 est destinée à ceinturer le tube interne servant de logement au système optique de transport d'image de l'endoscope. Un doigt radial 71 circulant dans la fente longitudinale 68 et vissable dans la partie distale du composant mécanique 69 permet d'associer de façon coulissante les composants 66 et 69.

Le composant mécanique 204 est une bague cylindrique destinée à être logée de façon coulissante dans la partie centrale du tube 18 solidaire de la sonde endoscopique et logé de façon tournante dans la poignée de commande 9 de ladite sonde. La bague 204 est dimensionnée pour ceinturer la partie distale du composant mécanique 66. Un doigt radial fileté 65 vissé dans la partie proximale de la bague 204 de façon à ce que l'extrémité dudit doigt puisse venir circuler dans la fente hélicoïdale 67 ménagée dans la partie distale du tube 66, permet d'associer les composants 66 et 204 de façon tournante et coulissante.

La partie proximale 73 du composant cylindrique 203 dispose de deux doigts radiaux externes 213 destinés à entraîner le déplacement longitudinal dudit composant dans le tube 18 solidaire de la sonde endoscopique, et ce grâce à un dispositif identique à celui décrit dans le texte relatif à la figure I.

La partie distale du composant cylindrique 204 dispose de deux doigts radiaux externes 214 destinés à entraîner le déplacement longitudinal dudit composant dans le tube 18 solidaire de la sonde endoscopique, et ce grâce à un dispositif identique à celui décrit dans le texte relatif à la figure I.

### FIGURE VII

La figure VII illustre la structure des dispositifs de commande intégrés dans la poignée 9 d'un endoscope à visée déviée relevant de la présente invention et bénéficiant simultanément des dispositifs suivants :
. Rotation de l'axe de visée autour de l'axe 1 de l'endoscope
. Rotation d'un réticule
. Réglage de mise au point.

Un dispositif de commande identique à celui décrit dans le texte relatif à la figure I agit sur les deux doigts radiaux 213 diamétralement opposés dont dispose la monture cylindrique 203 dans laquelle est fixement logée la lentille oculaire de l'endoscope, et ce de façon à entraîner le déplacement longitudinal de ladite monture.

Le doigt d'assemblage 72, vissé dans l'extrémité proximale de la monture cylindrique 69 dont l'orifice distal, faisant office de diaphragme de champ, sert également de logement à un support transparent sur lequel est gravé le réticule de l'endoscope, permet d'associer de façon tournante la monture 69 ceinturant l'extrémité distale de la monture 203 et ladite monture 203.

Le doigt d'assemblage 71, vissé dans l'extrémité distale de la monture cylindrique 69 et circulant dans la fente longitudinale 68 ménagée dans la partie proximale du tube cylindrique 66, permet d'associer de façon coulissante le tube 66 ceinturant l'extrémité distale de la monture 69 et ladite monture 69.

Le doigt d'assemblage 65, vissé dans l'extrémité proximale de la bague cylindrique 204 et circulant dans la fente hélicoïdale 67 ménagée dans la partie distale du tube 66, permet d'associer de façon tournante et coulissante la bague 204 ceinturant la partie distale du tube 66 et ladite monture 66. Un dispositif de commande identique à celui décrit dans le texte relatif à la figure I agit sur les deux doigts radiaux 214 diamétralement opposés dont dispose la bague 204, et ce de façon à entraîner le déplacement longitudinal de ladite bague.

### FIGURE VIII

La figure VIII illustre la structure d'un endoscope à visée déviée relevant de la présente invention et bénéficiant simultanément des dispositifs suivants.
. Rotation de l'axe de visée autour de l'axe 1 de l'endoscope
. Rotation d'un réticule 70 positionné dans le plan focal distal de la lentille oculaire 6.
. Réglage de mise au point

La structure mécanique de la sonde endoscopique est organisée autour d'un tube métallique externe 28 fixement solidaire d'un tube interne 29. L'extrémité proximale du tube externe 28 de la sonde endoscopique est fixement solidaire d'une bague de commande de rotation 30 ceinturant de façon tournante l'extrémité distale de la poignée cylindrique 9 de l'endoscope. Un dispositif de butée limitant la plage de rotation de la sonde endoscopique est constitué d'un doigt longitudinal 31 solidaire de la face proximale de la bague 30 et disposé de façon à venir en butée sur un doigt radial interne 32 solidaire de la poignée 9. La face proximale de la bague de commande de rotation 30 est fixement solidaire de l'extrémité distale d'un tube cylindrique 18 logé de façon tournante dans la poignée cylindrique 9 de l'endoscope et dont l'extrémité proximale tourne librement dans un palier 41 ménagé à cet effet à l'intérieur de l'extrémité proximale de ladite poignée.

Le dispositif optique de l'endoscope est constitué d'un hublot latéral 2 intégré dans l'extrémité distale du tube externe 28 de la sonde endoscopique, d'un prisme déviateur à réflexion partielle 3, d'un objectif constitué de plusieurs lentilles 4 logées dans l'extrémité distale du tube interne 29 de l'endoscope, d'un système optique de transport d'image constitué d'une série de lentilles achromatiques 5 disposées dans le tube interne 29 de l'endoscope, d'une lentille oculaire 6 logée dans la monture coulissante 203, d'un prisme correcteur 7 fixement logé dans l'extrémité proximale du tube cylindrique 18 et radialement positionné de façon à compenser l'inversion unidirectionnelle d'image introduite par le prisme déviateur distal 3, et d'une glace de protection 8 fixement logée dans l'extrémité proximale de la poignée cylindrique 9.

Le dispositif d'illumination intégré dans l'endoscope comprend un faisceau 25 de fibres optiques d'éclairage logé dans le volume annulaire compris entre le tube externe 28 et le tube interne 29 de la sonde endoscopique. L'extrémité distale du faisceau de fibres 25 est collée puis polie dans la fenêtre d'illumination latérale 24 ménagée dans l'extrémité distale du tube externe 28 de la sonde endoscopique. La partie proximale du faisceau de fibres 25 débouche dans le volume annulaire compris entre le tube 18 et la partie distale de la poignée cylindrique 9 à travers une fenêtre 34 ménagée dans la partie distale du tube cylindrique 18. L'extrémité proximale du faisceau de fibres 25 est collée puis polie dans l'embase latérale 33 fixement solidaire de la partie distale de la poignée 9. Plusieurs spires du faisceau de fibres d'éclairage 25 sont enroulées autour du tube 18 dans le volume annulaire compris entre ledit tube et la poignée cylindrique afin d'éviter qu'une rotation de 360° de la sonde endoscopique n'entraîne des contraintes mécaniques dangereuses sur ledit faisceau de fibres.

Un premier dispositif de commande analogue au dispositif 10 précédemment décrit dans le texte relatif à la figure I est intégré dans la partie proximale de la poignée de commande 9. La rotation de la bague de commande externe 123 dudit dispositif entraînant le déplacement longitudinal de la bague 143 ceinturant le tube 18, déplacement qui entraîne lui-même le déplacement longitudinal à l'intérieur du tube cylindrique 18 de la monture cylindrique 203 dans laquelle est fixement logée la lentille oculaire 6. Ladite monture 203 étant associée de façon tournante à une monture cylindrique 69 présentant un diamètre externe analogue à celui du tube 29 de la sonde endoscopique et dont l'orifice distal 39, faisant office de diaphragme de champ, sert de logement à un support transparent 70 sur lequel est gravé le réticule de l'endoscope. Ladite monture 69 venant coiffer l'extrémité distale de la monture 203 de façon à ce que le réticule 70 reste positionné en permanence dans le plan focal distal de la lentille oculaire 6.

Un second dispositif de commande analogue au dispositif 10 précédemment décrit dans le texte relatif à la figure I est intégré dans la partie médiane de la poignée de commande 9. La rotation de la bague de commande externe 124 dudit dispositif entraînant le déplacement longitudinal de la bague 144 ceinturant le tube 18, déplacement qui entraîne lui-même le déplacement longitudinal à l'intérieur du tube cylindrique 18 d'une bague cylindrique 204.

Un tube cylindrique 66 associe la bague cylindrique 204 et la monture porte réticule 69 conformément aux dispositions précédemment décrites dans les textes relatifs aux figures VI et VII. Ledit tube 66 ceinturant la monture porte réticule 69 ainsi que l'extrémité proximale du tube interne 29 de la sonde endoscopique. Ledit tube 66 étant lui-même ceinturé par la bague cylindrique 204. L'extrémité distale du tube 66 disposant d'une fente hélicoïdale dans laquelle circule un doigt solidaire de la bague 204, tandis que son extrémité proximale dispose d'une fente longitudinale dans laquelle circule un doigt solidaire de la monture porte réticule 69.

Les dispositifs de commande de rotation du réticule et de réglage de mise au point sont associés à un dispositif de rattrapage de jeu longitudinal constitué d'un ressort hélicoïdal 36 ceinturant la partie médiane du tube cylindrique 18. Ledit ressort étant comprimé entre la face proximale de la bague 144 du dispositif de commande de rotation de réticule et la face distale de la bague 143 du dispositif de commande de mise au point.

L'architecture cinématique décrite ci-dessus dans le texte relatif à la figure VII explicitant par ailleurs les dispositions suivantes :
. La monture cylindrique 203 de la lentille oculaire 6 est associée de façon tournante à la monture cylindrique 69 du réticule.
. La monture 69 du réticule est associée de façon coulissante à l'extrémité proximale d'un tube cylindrique 66 dont l'extrémité distale est quant à elle associée de façon tournante et coulissante à la bague cylindrique 204 par l'intermédiaire d'un doigt solidaire de ladite bague et circulant dans une fente hélicoïdale ménagée dans la partie distale dudit tube.
. La bague 204 ceinture le tube 66 qui ceinture lui-même l'extrémité proximale du tube interne 29 de la sonde endoscopique.

Ladite architecture cinématique interdisant toute interaction entre la rotation de la sonde commandée par la bague externe 32, la rotation du réticule commandée par la bague externe 124, et le déplacement longitudinal de l'oculaire 6 commandé par la bague externe 123.

### FIGURE IX

La figure IX illustre la structure d'un endoscope à visée déviée relevant de la présente invention et bénéficiant simultanément des dispositifs suivants :
. Rotation de l'axe de visée autour de l'axe 1 de l'endoscope.
. Variation de l'angle de visée de l'endoscope.
. Rotation d'un réticule 70 positionné dans le plan focal distal de la lentille oculaire 6.
. Réglage de mise au point.

La structure mécanique de la sonde endoscopique est organisée autour d'un tube métallique externe 28 dont l'extrémité distale est fixement solidaire d'un embout distal 46, d'un tube interne 29 fixement solidaire du tube externe 28, et d'un tube médian 27 ceinturant de façon coulissante le tube interne 29. L'extrémité proximale du tube externe 28 de la sonde endoscopique est fixement solidaire d'une bague de commande de rotation 30 ceinturant l'extrémité distale de la poignée cylindrique 9 de l'endoscope. Un dispositif de butée limitant la plage de rotation de la sonde endoscopique est constitué d'un doigt longitudinal 31 solidaire de la face proximale de la bague 30 et disposé de façon à venir en contact avec un doigt radial interne 32 solidaire de la poignée 9. La face proximale de la bague de commande de rotation 30 est fixement solidaire de l'extrémité distale d'un tube cylindrique 18 logé dans la poignée cylindrique 9 de l'endoscope et dont l'extrémité proximale tourne librement dans un palier 41 ménagé à cet effet à l'intérieur de l'extrémité proximale de ladite poignée.

Le dispositif optique de l'endoscope est constitué d'un hublot latéral 2 intégré dans l'embout distal 46, d'un prisme déviateur à réflexion partielle 3 également intégré dans l'embout distal 46, d'un objectif constitué de plusieurs lentilles 4 logées dans l'extrémité distale du tube interne 29, d'un système optique de transport d'image constitué d'une série de lentilles achromatiques 5 disposées dans le tube interne 29, d'une lentille oculaire 6 logée de façon coulissante dans le tube cylindrique 18, d'un prisme correcteur 7 fixement logé dans l'extrémité proximale du tube cylindrique 18 et radialement positionné de façon à compenser l'inversion unidirectionnelle d'image introduite par le prisme déviateur distal 3, et d'une glace de protection 8 fixement logée dans l'extrémité proximale de la poignée cylindrique 9.

Le dispositif d'illumination intégré dans l'endoscope comprend un faisceau 25 de fibres optiques d'éclairage logé dans le volume annulaire compris entre le tube coulissant 27 et le tube externe 28. L'extrémité distale du faisceau de fibres 25 est divisée en trois sous-faisceaux dont les extrémités distales sont collées puis polies dans trois fenêtres d'illumination latérale 22/23/24 ménagées dans l'embout distal 46. Les directions desdites fenêtres étant calculées de telle façon que la fenêtre 24 corresponde à une illumination latérale, la fenêtre 22 à une illumination rétrograde, et la fenêtre 23 à une illumination prograde. La partie proximale du faisceau de fibres 25 débouche dans le volume annulaire compris entre le tube 18 et la partie distale de la poignée cylindrique 9 à travers une fenêtre 34 ménagée dans la partie distale du tube cylindrique 18. L'extrémité proximale du faisceau de fibres 25 est collée puis polie dans l'embase latérale 33 fixement solidaire de la partie distale de la poignée 9. Plusieurs spires du faisceau de fibres 25 sont enroulées autour du tube cylindrique 18 dans le volume annulaire compris entre ledit tube et la poignée cylindrique afin d'éviter qu'une rotation de 360° de la sonde endoscopique n'entraîne des contraintes mécaniques dangereuses sur ledit faisceau de fibres.

Un premier dispositif de commande analogue au dispositif 10 précédemment décrit dans le texte relatif à la figure I est intégré dans la partie proximale de la poignée de commande 9. La rotation de la bague de commande externe 123 dudit dispositif entraînant le déplacement longitudinal de la bague 143 ceinturant le tube 18, déplacement qui entraîne lui-même le déplacement longitudinal à l'intérieur du tube cylindrique 18 de la monture cylindrique 203 dans laquelle est fixement logée la lentille oculaire 6. Ladite monture 203 étant associée de façon tournante à une monture cylindrique 69 présentant un diamètre externe analogue à celui du tube 29 de la sonde endoscopique et dont l'orifice distal 39, faisant office de diaphragme de champ, sert de logement à un support transparent 70 sur lequel est gravé le réticule de l'endoscope. Ladite monture 69 venant coiffer l'extrémité distale de la monture 203 de façon à ce que le réticule 70 reste positionné en permanence dans le plan focal distal de la lentille oculaire 6.

Un second dispositif de commande analogue au dispositif 10 précédemment décrit dans le texte relatif à la figure I est intégré dans la partie médiane de la poignée de commande 9. La rotation de la bague de commande externe 124 dudit dispositif entraînant le déplacement longitudinal de la bague 144 ceinturant le tube 18, déplacement qui entraîne lui-même le déplacement longitudinal à l'intérieur du tube cylindrique 18 d'une bague cylindrique 204.

Un tube cylindrique 66 associe la bague cylindrique 204 et la monture porte réticule 69 conformément aux dispositions précédemment décrites dans les textes relatifs aux figures VI et VII. Ledit tube 66 ceinturant la monture porte réticule 69 ainsi que l'extrémité proximale du tube interne 29 de la sonde endoscopique. Ledit tube 66 étant lui-même ceinturé par la bague cylindrique 204. L'extrémité distale du tube 66 disposant d'une fente hélicoïdale dans laquelle circule un doigt solidaire de la bague 204, tandis que son extrémité proximale dispose d'une fente longitudinale dans laquelle circule un doigt solidaire de la monture porte réticule 69.

Un troisième dispositif de commande analogue au dispositif 10 précédemment décrit dans le texte relatif à la figure I est intégré dans la partie distale de la poignée de commande 9. La rotation de la bague de commande externe 122 dudit dispositif entraînant le déplacement longitudinal de la bague 142, déplacement qui entraîne lui-même le déplacement longitudinal à l'intérieur du tube 18 de la monture cylindrique 202 ceinturant fixement l'extrémité proximale du tube de manoeuvre 27 coulissant autour du tube interne 29. Le déplacement longitudinal de la bille sphérique 45 fixement solidaire de l'ergot longitudinal 44 constituant l'extrémité distale du tube 27 entraînant le basculement autour de l'axe transversal 63 du support mécanique 54 du prisme déviateur 3. Ledit support 54 étant logé dans un étrier 59 dont l'extrémité distale 62 est fixement solidaire de la branche longitudinale 53 du support en forme d'équerre disposé à l'intérieur de l'embout distal 46.

Le dispositif de réglage de mise au point de l'endoscope est associé à un dispositif de rattrapage de jeu longitudinal constitué d'un ressort hélicoïdal 38 ceinturant l'extrémité proximale du tube cylindrique 18. L'extrémité distale dudit ressort venant en appui sur la face proximale de la bague folle 143 ceinturant le tube 18 tandis que son extrémité proximale vient en appui sur la face distale du palier ménagé dans l'extrémité proximale de la poignée 9.

Les dispositifs de commande de rotation du réticule et de commande de variation de l'angle de visée sont associés à un dispositif de rattrapage de jeu longitudinal constitué d'un ressort hélicoïdal 37 ceinturant la partie médiane du tube cylindrique 18. Ledit ressort étant comprimé entre la face proximale de la bague 142 du dispositif de commande de variation de l'angle de visée de l'endoscope et la face distale de la bague 144 du dispositif de commande de rotation du réticule.

### PORTEE DE L'INVENTION

II va de soi que les applications des endoscopes rotatifs à visée distale déviée faisant l'objet de la présente invention peuvent être aussi bien médicales qu'industrielles.

Il va également de soi que la présente invention ne se limite nullement aux modes de réalisation et de mise en oeuvre qui viennent d'être décrits de façon explicite. La présente invention embrasse au contraire toutes les variantes qui peuvent venir à l'esprit du technicien en la matière sans s'écarter pour autant du cadre de la présente invention, et notamment des endoscopes dont les dispositifs de captation et/ou de transport d'image font appel à des technologies différentes de celles évoquées dans le cadre de la présente invention.

## Revendications

1. Endoscope rotatif à visée distale déviée comprenant :
. une poignée tubulaire cylindrique (9),
. une sonde comprenant un tube cylindrique interne (29) fixement associé à un tube cylindrique externe (28) dont l'extrémité proximale est fixement solidaire d'une bague de commande de rotation de l'axe de visée (30), entraînant la sonde en rotation axiale et ceinturant de façon tournante l'extrémité distale de la poignée (9), ladite bague étant fixement solidaire d'un tube cylindrique tournant (18) logé de façon tournante dans la poignée (9), le prolongement proximal du tube interne (29) débouchant dans le tube tournant (18),
. un dispositif optique comportant un hublot latéral (2) intégré dans la partie distale du tube externe (28), un prisme déviateur (3) et un objectif logés dans la partie distale du tube interne (29), un système optique de transport d'image (5) logé dans le tube interne (29), un oculaire (6) disposé dans le tube tournant (18) et un hublot d'observation (8) fermant l'orifice proximal de la poignée (9),
**caractérisé en ce que** la poignée (9) comprend plusieurs dispositifs de commande (10) totalement indépendants, chacun desdits dispositifs comprenant une bague de commande (12) ceinturant de façon tournante la poignée (9), une monture cylindrique (20) logée de façon coulissante dans le tube tournant (18), et un système d'accouplement associant la bague de commande (12) à la monture cylindrique (20), de manière à ce qu'un mouvement de rotation de la bague de commande (12) entraîne un mouvement de translation longitudinal de la monture (20) à l'intérieur du tube tournant (18), le mouvement de rotation du tube tournant (18) et les mouvements de rotation de la bague de commande (12) et de translation de la monture (20) de chacun desdits dispositifs de commande étant indépendants les uns des autres.

2. Endoscope rotatif à visée distale déviée selon la revendication 1, **caractérisé en ce que** la monture tubulaire cylindrique (20) de chacun des dispositifs de commande (10) comprend au moins un doigt radial externe (21) logé de façon coulissante dans une fente longitudinale (19) ménagée dans le tube tournant (18), l'extrémité du doigt (21) de la monture étant logée de façon circulante dans une gorge annulaire interne (16) ménagée dans une bague coulissante (14) ceinturant de façon coulissante le tube tournant (18) et logée de façon coulissante dans la poignée (9), ladite bague coulissante (14) disposant d'un doigt radial externe (17) logé de façon coulissante dans une fente longitudinale (11) ménagée dans la poignée (9), l'extrémité du doigt (17) de la bague étant logée de façon circulante dans une gorge hélicoïdale interne (13) ménagée dans la bague de commande (12).

3. Endoscope rotatif à visée distale déviée selon les revendications 1 ou 2 **caractérisé en ce que** l'un desdits dispositifs (10) de commande est un dispositif de commande de mise au point comportant une monture tubulaire cylindrique (201) logeant fixement l'oculaire (6).

4. Endoscope rotatif à visée distale déviée selon la revendication 3, **caractérisé en ce que** la monture tubulaire cylindrique (201) du dispositif de commande de mise au point présente un orifice distal (39) positionné dans le plan focal distal de l'oculaire (6) et faisant office de diaphragme de champ.

5. Endoscope rotatif à visée distale déviée selon la revendication 3 ou 4, **caractérisé en ce que** l'un desdits dispositifs de commande (10) est un dispositif de commande de rotation d'un réticule, la monture (203) du dispositif de réglage de mise au point présentant une partie tubulaire cylindrique distale (74) d'un diamètre externe inférieur à celui de sa partie proximale (73), le dispositif de commande de rotation d'un réticule comprenant :
un tube cylindrique support de réticule (69) ceinturant la partie distale (74) de la monture (203) du dispositif de réglage de mise au point, et présentant un diamètre externe sensiblement identique à celui du tube interne (29) et un orifice distal (39) faisant office de diaphragme de champ et logeant un réticule (70) de telle façon que ledit réticule reste positionné en permanence dans le plan focal distal de l'oculaire (6),
une bague de commande (124) ceinturant de façon tournante la partie centrale de la poignée (9),
une monture tubulaire cylindrique (204) logée de façon coulissante dans le tube tournant (18) et entraînée longitudinalement par la rotation de la bague de commande (124) du dispositif de commande de rotation du réticule,
un tube cylindrique de couplage (66) associant la monture (204) au tube support de réticule (69) et présentant une partie proximale ceinturant le tube support de réticule (69) et une partie distale ceinturant l'extrémité proximale du tube interne (29) et ceinturée par la monture (204) du dispositif de commande de rotation du réticule,
un système d'accouplement associant en rotation le tube de couplage (66) au tube support de réticule (69), et
un système d'accouplement associant le tube de couplage (66) à la monture (204) du dispositif de commande de rotation du réticule, de manière à ce qu'un mouvement longitudinal de la monture (204) du dispositif de commande de rotation du réticule entraîne un mouvement de rotation du tube de couplage (66) et donc un mouvement de rotation du tube (69) support de réticule.

6. Endoscope rotatif à visée distale déviée selon la revendication 5, **caractérisé en ce que** la partie tubulaire distale (74) de la monture (203) du dispositif de commande de mise au point présente une gorge annulaire externe (75) dans laquelle est logé de façon circulante l'extrémité d'un doigt radial interne (72) formé dans la partie proximale du tube support de réticule (69), la partie proximale du tube de couplage (66) comprenant une fente longitudinale (68) dans laquelle est logé de façon circulante un doigt (71) radial externe formé sur la partie distale du tube support de réticule (69), la partie distale du tube de couplage (66) disposant d'une fente hélicoïdale (67) dans laquelle est logé de façon circulante un doigt externe (65) formé sur la monture (204) du dispositif de commande de rotation du réticule.

7. Endoscope rotatif à visée distale déviée selon l'une quelconque des précédentes revendications, **caractérisé en ce que** l'un des dispositifs de commande (10) intégrés dans la poignée (9) dudit endoscope est un dispositif de commande de variation d'angle de visée commandé par une bague (122) ceinturant de façon tournante la partie distale de la poignée (9) et dont la rotation entraîne un déplacement longitudinal d'une monture tubulaire cylindrique (202) logée de façon coulissante dans le tube tournant (18) et ceinturant de façon coulissante le prolongement proximal du tube interne (29), la monture (202) constituant l'extrémité proximale d'un tube cylindrique de manoeuvre (27) ceinturant de façon coulissante le tube interne (29), le déplacement longitudinal dudit tube de manoeuvre (27) entraînant un basculement du prisme déviateur (3) autour d'un axe (64) perpendiculaire à l'axe de visée de l'endoscope.

8. Endoscope rotatif à visée distale déviée selon la revendication 7, **caractérisé en ce que** :
l'extrémité distale de la sonde endoscopique est constituée d'un embout (46) présentant une face transversale distale (48) fixement associée à l'extrémité distale (43) du tube externe (28), et une face longitudinale plate (47) fixement associée à une échancrure longitudinale (42) ménagée dans la partie distale dudit tube externe,
l'embout distal (46) comprend une protubérance interne en forme d'équerre dont la partie longitudinale (53) est fixement associée à l'extrémité distale (62) d'une pièce (59) dont la partie proximale est constituée de deux oreilles longitudinales (60) en regard l'une de l'autre,
le prisme déviateur (3) est fixement logé dans un support (54) de prisme fixé entre les deux oreilles (60) de la pièce (59) de manière à pouvoir basculer autour d'un axe transversal (64) perpendiculaire à l'axe de visée de l'endoscope,
le support (54) est couplé à l'extrémité distale du tube de manoeuvre (27) de telle façon qu'un déplacement longitudinal dudit tube de manoeuvre entraîne un basculement du support de prisme (54) autour de l'axe transversal (64).

9. Endoscope rotatif à visée distale déviée suivant l'une quelconque des précédentes revendications **caractérisé en ce que** :
le prisme déviateur distal (3) est un prisme à réflexion partielle réalisant une inversion unidirectionnelle de l'image transmise par ledit prisme,
un prisme correcteur (7) à réflexion partielle réalisant une inversion unidirectionnelle de l'image transmise par ledit prisme correcteur est fixement logé dans l'extrémité proximale du tube tournant (18) et radialement positionné de façon à ce que l'image transmise par ledit prisme correcteur (5) ne présente aucune inversion par rapport à la réalité.

10. Endoscope rotatif à visée distale déviée suivant l'une quelconque des revendications 1 à 8 **caractérisé en ce que** :
le prisme déviateur distal (3) est un prisme à réflexion partielle **caractérisé par** une inversion unidirectionnelle de l'image transmise par ledit prisme.
un prisme correcteur à réflexion partielle **caractérisé par** une inversion unidirectionnelle de l'image transmise par ledit prisme correcteur est fixement logé entre deux lentilles (5) dans le tube interne (29) et radialement positionné de façon à ce que l'image transmise par la lentille oculaire (6) ne présente aucune inversion par rapport à la réalité.

11. Endoscope rotatif à visée distale déviée suivant l'une quelconque des revendications 1 à 8 **caractérisé en ce que** :
le prisme déviateur,distal (3) est un prisme à réflexion totale **caractérisé par** une inversion bidirectionnelle de l'image transmise par ledit prisme.
le train de lentilles (5) constituant le système de transport optique d'image est calculé de façon à ce que l'image transmise par la lentille oculaire (6) ne présente aucune inversion par rapport à la réalité.

12. Endoscope rotatif à visée distale déviée selon l'une quelconque des précédentes revendications, **caractérisé en ce qu'**il comprend un dispositif de butée destiné limiter la plage de rotation de la bague (30) de commande rotation de l'axe de visée.

13. Endoscope rotatif à visée distale déviée selon la revendication 12, **caractérisé en ce que** ce que le dispositif de butée comprend un doigt longitudinal (31) solidaire de la face proximale de la bague (30) de commande de rotation de l'axe de visée, ledit doigt débouchant à l'intérieur de la poignée cylindrique (9) de façon à pouvoir venir en contact avec un doigt radial interne (32) solidaire de ladite poignée.

14. Endoscope rotatif à visée distale déviée selon la revendication 12 ou 13, **caractérisé en ce qu'**il comprend un dispositif d'éclairage constitué d'un faisceau (25) de fibres optiques logé dans le volume annulaire compris entre les tubes interne et externe (28, 29), l'extrémité distale dudit faisceau débouchant dans au moins une fenêtre d'illumination ménagée dans la partie distale du tube externe (28) de manière à ce que le champ d'illumination recouvre le champ optique de la sonde, la partie proximale du faisceau de fibres (25) débouchant à l'intérieur de la poignée (9) à travers un orifice (34) ménagé dans la partie distale du tube tournant (18).

15. Endoscope rotatif à visée distale déviée selon la revendication 12, **caractérisé en ce que** la partie proximale du faisceau de fibres (25) est lovée autour du tube tournant (18) afin d'éviter que la rotation de la sonde n'entraîne de contraintes susceptibles de briser les fibres dudit faisceau.

## Claims

1. A rotary deflected distal view endoscope comprising:
- a cylindrical tubular handle (9),
- a probe comprising an inner cylindrical tube (29) fixedly associated with an outer cylindrical tube (28), the proximal end of which is fixedly connected to a ring controlling the rotation of the viewing axis (30), driving the probe in axial rotation and rotatably surrounding the distal end of the handle (9), the said ring being fixedly connected to a rotating cylindrical tube (18) housed rotatably in the handle (9), the proximal prolongation of the inner tube (29) leading into the rotating tube (18),
- an optical device comprising a lateral hatch (2) integrated in the distal part of the outer tube (28), a deflector prism (3) and an objective housed in the distal part of the inner tube (29), an optical image transport system (5) housed in the inner tube (29), an eyepiece (6) disposed in the rotating tube (18) and an observation hatch (8) forming the proximal orifice of the handle (9),
**characterised in that** the handle (9) comprises a plurality of totally independent control devices (10), each of said devices comprising a control ring (12) rotatably surrounding the handle (9), a cylindrical mount (20) housed slidably in the rotating tube (18), and a coupling system associating the control ring (12) with the cylindrical mount (20) so that a rotary movement of the control ring (12) results in a movement of longitudinal translation of the mount (20) inside the rotating tube (18), the rotary movement of the rotating tube (18) and the rotary movement of the control ring (12) and the translatory movement of the mount (20) of each of the said control devices being independent of one another.

2. A rotary deflected distal view endoscope according to claim 1, **characterised in that** the cylindrical tubular mount (20) of each of the control devices (10) comprises at least one outer radial finger (21) housed slidably in a longitudinal slot (10) formed in the rotating tube (18), the end of the finger (21) of the mount being housed so as to circulate in an inner annular groove (16) formed in a sliding ring (14) slidably surrounding the rotating tube (18) and housed slidably in the handle (9), the said sliding ring (14) having an outer radial finger (17) housed slidably in a longitudinal slot (11) formed in the handle (9), the end of the finger (17) of the ring being housed so as to circulate in an inner helical groove (13) formed in the control ring (12).

3. A rotary deflected distal view endoscope according to claim 1 or 2, **characterised in that** one of the said control devices (10) is a focusing control device comprising a cylindrical tubular mount (201) fixedly housing the eyepiece (6).

4. A rotary deflected distal view endoscope according to claim 3, **characterised in that** the cylindrical tubular mount (201) of the focusing control device has a distal orifice (39) positioned in the distal focal plane of the eyepiece (6) and acting as a field diaphragm.

5. A rotary deflected distal view endoscope according to claim 3 or 4, **characterised in that** one of the said control devices (10) is a device for controlling rotation of a graticle, the mount (203) of the focusing adjustment device having a distal cylindrical tubular part (74) of an outside diameter less than that of its proximal part (73), the graticle rotation control device comprising:
a cylindrical graticle support tube (69) surrounding the distal part (74) of the mount (203) of the focusing adjustment device, and having an outside diameter substantially identical to that of the inner tube (29) and a distal orifice (39) acting as a field diaphragm and housing a graticle (70) in such manner that said graticle remains permanently positioned in the distal focal plane of the eyepiece (6),
a control ring (124) rotatably surrounding the central part of the handle (9),
a cylindrical tubular mount (204) housed slidably in the rotating tube (18) and driven longitudinally by the rotation of the control ring (124) of the graticle rotation control device,
a cylindrical coupling tube (66) associating the mount (204) with the graticle support tube (69) and having a proximal part surrounding the graticle support tube (69) and the distal part surrounding the proximal end of the inner tube (29) and surrounded by the mount (204) of the graticle rotation control device,
a coupling system rotatably associating the coupling tube (66) with the graticle support tube (69), and
a coupling system associating the coupling tube (66) with the mount (204) of the graticle rotation control device in such manner that a longitudinal movement of the mount (204) of the graticle rotation control device results in a rotary movement of the coupling tube (66) and hence a rotary movement of the graticle support tube (69).

6. A rotary deflected distal view endoscope according to claim 5, **characterised in that** the distal tubular part (74) of the mount (203) of the focusing control device has an outer annular groove (75) in which there is housed so as to circulate the end of an inner radial finger (72) formed in the proximal part of the graticle support tube (69), the proximal part of the coupling tube (66) comprising a longitudinal slot (68) in which there is housed in circulating manner an outer radial finger (71) formed on the distal part of the graticle support tube (69), the distal part of the coupling tube (66) having a helical slot (67) in which there is housed in circulating manner an outer finger (65) formed on the mount (204) of the graticle rotation control device.

7. A rotary deflected distal view endoscope according to any one of the preceding claims, **characterised in that** one of the control devices (10) integrated in the handle (9) of said endoscope is a device for controlling the variation of the viewing angle controlled by a ring (122) rotatably surrounding the distal part of the handle (9) and the rotation of which results in a longitudinal movement of a cylindrical tubular mount (202) housed slidably in the rotating tube (18) and slidably surrounding the proximal prolongation of the inner tube (29), the mount (202) forming the proximal end of a cylindrical manipulating tube (27) slidably surrounding the inner tube (29), the longitudinal movement of said manipulating tube (27) resulting in a pivoting of the deflector prisms (3) about an axis (64) perpendicular to the viewing axis of the endoscope.

8. A rotary deflected distal view endoscope according to claim 7, **characterised in that**:
the distal end of the endoscope probe consists of an end cap (46) having a distal transverse surface (48) fixedly associated with the distal end (43) of the outer tube (28), and a flat longitudinal surface (47) fixedly associated with a longitudinal notch (42) formed in the distal part of said outer tube,
the distal end cap (46) comprises a square shaped inner protuberance of which the longitudinal part (53) is fixedly associated with the distal end (62) of a part (59) of which the proximal part is formed by two longitudinal lugs (60) opposite one another,
the deflector prism (3) is fixedly housed in a prism support (54) fixed between the two lugs (60) of the part (59) so as to be able to pivot about a transverse axis (64) perpendicular to the viewing axis of the endoscope,
the support (54) is coupled to the distal end of the manipulating tube (27) in such manner that a longitudinal movement of said manipulating tube results in pivoting of the prism support (54) about the transverse axis (64).

9. A rotary deflected distal view endoscope according to any one of the preceding claims, **characterised in that**:
the distal deflector prisms (3) is a partial reflection prism effecting a unidirectional inversion of the image transmitted by said prism,
a partial reflection corrector prism (7) effecting a unidirectional inversion of the image transmitted by said corrector prism is fixedly housed in the proximal end of the rotating tube (18) and positioned radially in such manner that the image transmitted by said corrector prism (5) has no inversion in relation to reality.

10. A rotary deflected distal view endoscope according to any one of claims 1 to 8, **characterised in that**
the distal deflector prism (3) is a partial reflection prism **characterised by** a unidirectional inversion of the image transmitted by said prism,
a partial reflection corrector prism **characterised by** a unidirectional inversion of the image transmitted by said corrector prism is fixedly housed between two lens elements (5) in the inner tube (29) and radially positioned in such manner that the image transmitted by the ocular lens element (6) has no inversion in relation to reality.

11. A rotary deflected distal view endoscope according to any one of claims 1 to 8, **characterised in that**
the distal deflector prism (3) is a total reflection prism **characterised by** a bi-directional inversion of the image transmitted by said prism,
the train of lens elements (5) forming the optical image transport system is so designed that the image transmitted by the ocular lens element (6) has no inversion in relation to reality.

12. A rotary deflected distal view endoscope according to any one of the preceding claims, **characterised in that** it comprises a stop device intended to limit the range of rotation of the ring (30) controlling the rotation of the viewing axis.

13. A rotary deflected distal view endoscope according to claim 12, **characterised in that** the stop device comprises a longitudinal finger (31) connected to the proximal surface of the ring (30) controlling the rotation of the viewing axis, said ring leading to the interior of the cylindrical handle (9) so as to be able to come into contact with an inner radial finger (32) connected to said. handle.

14. A rotary deflected distal view endoscope according to claim 12 or 13, **characterised in that** it comprises a lighting device consisting of a bundle (25) of optical fibres housed in the annular volume contained between the inner and outer tubes (28, 29), the distal end of said bundle leading into at least one illumination window formed in the distal part of the outer tube (28) in such manner that the field of illumination covers the optical field of the probe, the proximal part of the fibre bundle (25) leading to the interior of the handle (9) through an orifice (34) formed in the distal part of the rotating tube (18).

15. A rotary deflected distal view endoscope according to claim 12, **characterised in that** the proximal part of the fibre bundle (25) is raised around the rotating tube (18) to prevent rotation of the probe from producing stresses capable of breaking the fibres of said bundle.

## Patentansprüche

1. Dreh-Endoskop mit abgelenkter Femvisur, das Folgendes umfasst:
. einen zylindrischen Hülsengriff (9),
. eine Sonde mit einer inneren zylindrischen Hülse (29), welche untrennbar mit einer äußeren zylindrischen Hülse (28) verbunden ist, deren proximales Ende untrennbar mit einem die Drehung der Visierachse steuernden Ring (30) verbunden ist, der die Sonde in eine Drehbewegung um die Achse versetzt und das distale Ende des Griffs (9) drehend umgibt, wobei der besagte Ring untrennbar mit einer drehenden zylindrischen Hülse (18) verbunden ist, die drehbar im Griff (9) untergebracht ist, wobei der proximale Fortsatz der inneren Hülse (29) in die drehende Hülse (18) einmündet,
. eine optische Vorrichtung, die ein seitliches, in den distalen Teil der äußeren Hülse (28) integriertes Fenster (2), ein Ablenkprisma (3) und ein Objektiv, die im distalen Teil der inneren Hülse (29) untergebracht sind, ein in der inneren Hülse (29) untergebrachtes optisches Bildtransportsystem (5), ein in der drehenden Hülse (18) angeordnetes Okular (6) und ein Sichtfenster (8) umfasst, das die proximale Öffnung des Griffs (9) schließt,
**dadurch gekennzeichnet, dass** der Griff (9) mehrere vollkommen unabhängige Steuervorrichtungen (10) aufweistt, wobei jede der besagten Vorrichtungen einen den Griff (9) drehend umgebenden Steuerring (12), eine verschiebbar in der drehenden Hülse (18) untergebrachte zylindrische Fassung (20) und ein Kupplungssystem aufweist, welches den Steuerring (12) mit der zylindrischen Fassung (20) verbindet, sodass eine Drehbewegung des Steuerrings (12) eine Translationsbewegung in Längsrichtung der Fassung (20) im Innern der drehenden Hülse (18) zur Folge hat, wobei die Drehbewegung der drehenden Hülse (18) sowie die Drehbewebung des Steuerrings (12) und die Translationsbewegung der Fassung (20) jeder der besagten Steuervorrichtungen voneinander unabhängig sind.

2. Dreh-Endoskop mit abgelenkter Fernvisur gemäß Anspruch 1, **dadurch gekennzeichnet, dass** die zylindrische Hülsenfassung (20) jeder der Steuervorrichtungen (10) mindestens einen äußeren Radialstift (21) aufweist, der verschiebbar in einem in der drehenden Hülse (18) angelegten Längsschlitz (19) untergebracht ist, wobei das Ende des Stifts (21) der Fassung umlaufend in einer inneren Ringnut (16) untergebracht ist, die in einem verschiebbaren Ring (14) angelegt ist, der die drehende Hülse (18) verschiebbar umgibt und verschiebbar im Griff (9) untergebracht ist, wobei der besagte verschiebbare Ring (14) über einen äußeren Radialstift (17) verfügt, der verschiebbar in einem im Griff (9) angelegten Längsschlitz (11) untergebracht ist, wobei das Ende des Stifts (17) des Rings umlaufend in einer inneren, im Steuerring (12) angelegten spiralförmigen Nut (13) untergebracht ist.

3. Dreh-Endoskop mit abgelenkter Fernvisur gemäß Anspruch 1 oder 2, **dadurch gekennzeichnet, dass** eine der besagten Steuervorrichtungen (10) eine Steuervorrichtung für die Fokussierung ist, die eine zylindrische Hülsenfassung (201) besitzt, die das Okular (6) fest einschließt.

4. Dreh-Endoskop mit abgelenkter Fernvisur gemäß Anspruch 3, **dadurch gekennzeichnet, dass** die zylindrische Hülsenfassung (201) der Steuervorrichtung für die Fokussierung eine distale Öffnung (39) aufweist, die in der distalen Brennpunktebene des Okulars (6) positioniert ist und als Sehfeldblende dient.

5. Dreh-Endoskop mit abgelenkter Fernvisur gemäß Anspruch 3 oder 4, **dadurch gekennzeichnet, dass** eine der besagten Steuervorrichtungen (10) eine Steuervorrichtung für die Drehung eines Fadenkreuzes ist, wobei die Fassung (203) der Regelungsvorrichtung für die Fokussierung einen distalen zylindrischen Hülsenteil (74) mit einem Außendurchmesser kleiner als der seines proximalen Teils (73) aufweist, und die Steuervorrichtung für die Fadenkreuzdrehung Folgendes umfasst:
eine zylindrische Fadenkreuzträgerhülse(69), die den distalen Teil (74) der Fassung (203) der Regelungsvorrichtung für die Fokussierung umgibt, und die einen mit dem der inneren Hülse (29) in etwa identischen Außendurchmesser und eine distale Öffnung (39) aufweist, welche als Sehfeldblende dient und in der ein Fadenkreuz (70) so untergebracht ist, dass das besagte Fadenkreuz ständig in der distalen Brennpunktebene des Okulars (6) positioniert bleibt,
einen Steuerring (124), der den mittleren Teil des Griffs (9) drehend umgibt,
eine zylindrische Hülsenfassung (204), die verschiebbar in der drehenden Hülse (18) untergebracht ist und durch die Drehbewegung des Steuerrings (124) der Steuerungsvorrichtung für die Fadenkreuzdrehung in Längsrichtung mitgeführt wird,
eine zylindrische Kopplungshülse (66), welche die Fassung (204) mit der Fadenkreuzträgerhülse (69) verbindet und einen proximalen Teil, der die Fadenkreuzträgerhülse (69) umgibt, sowie einen distalen Teil, der das proximale Ende der inneren Hülse (29) umgibt und von der Fassung (204) der Steuerungsvorrichtung für die Fadenkreuzdrehung umgeben ist, aufweist,
ein Kupplungssystem, das bei der Drehung dieKopplungshülse (66) mit der Fadenkreuzträgerhülse (69) verbindet, und
ein Kupplungssystem, das die Kopplungshülse (66) mit der Fassung (204) der Steuerungsvorrichtung für die Fadenkreuzsdrehung verbindet, sodass eine Längsbewegung der Fassung (204) der Steuerungsvorrichtung für die Fadenkreuzdrehung eine Drehbewegung der Kopplungshülse (66) und somit eine Drehbewegung der Fadenkreuzträgerhülse (69) mit sich bringt.

6. Dreh-Endoskop mit abgelenkter Fernvisur gemäß Anspruch 5, **dadurch gekennzeichnet, dass** der distale Hülsenteil (74) der Fassung (203) der Steuerungsvorrichtung für die Fokussierung eine äußere Ringnut (75) aufweist, in der das Ende eines inneren Radialstifts (72) umlaufend untergebracht ist, der im proximalen Teil der Fadenkreuzträgerhülse (69) geformt ist, wobei der proximale Teil der Kopplungshülse (66) einen Längsschlitz (68) besitzt, in dem ein äußerer Radialstift (71) umlaufend untergebracht ist, der auf dem distalen Teil der Fadenkreuzträgerhülse (69) geformt ist, und der distale Teil der Kopplungshülse (66) über einen spiralförmigen Schlitz (67) verfügt, in dem ein äußerer Stift (65) umlaufend untergebracht ist, der auf der Fassung (204) der Steuerungsvorrichtung für die Fadenkreuzdrehung geformt ist.

7. Dreh-Endoskop mit abgelenkter Fernvisur gemäß einem beliebigen der vorausgehenden Ansprüche, **dadurch gekennzeichnet, dass** eine der in den Griff (9) des besagten Endoskops integrierte Steuerungsvorrichtungen (10) eine Steuerungsvorrichtung für die Sichtwinkeländerung ist, die von einem Ring (122) gesteuert wird, der den distalen Teil des Griffs (9) umlaufend umgibt und dessen Drehung die Längsverschiebung einer zylindrischen Hülsenfassung (202) mit sich bringt, die verschiebbar in der drehenden Hülse (18) untergebracht ist und den proximalen Fortsatz der inneren Hülse (29) verschiebbar umgibt, wobei die Fassung (202) das proximale Ende einer zylindrischen Bedienungshülse (27) bildet, welche die innere Hülse (29) verschiebbar umgibt, und die Längsverschiebung der besagten Bedienungshülse (27) ein Kippen des Ablenkprismas (3) um eine Achse (64) senkrecht zur Visierachse des Endoskops zur Folge hat.

8. Dreh-Endoskop mit abgelenkter Femvisur gemäß Anspruch 7, **dadurch gekennzeichnet, dass**:
das distale Ende der Endoskop-Sonde aus einem Ansatzstück (46) besteht, das eine distale Querseite (48), die mit dem distalen Ende (43) der äußeren Hülse (28) untrennbar verbunden ist, und eine flache Längsseite (47), die mit einem bogenförmigen, im distalen Ende der besagten äußeren Hülse angelegen Längsausschnitt (42) untrennbar verbunden ist, aufweist,
das distale Ansatzstück (46) eine innere Ausstülpung in Winkelform besitzt, deren Längsteil (53) untrennbar mit dem distalen Ende (62) eines Bauteils (59) verbunden ist, dessen proximaler Teil aus zwei sich gegenüberliegenden Zapfen (60) besteht,
das Ablenkprisma (3) untrennbar in einem zwischen den beiden Zapfen (60) des Bauteils (59) befestigten Prisma-Träger (54) untergebracht ist, sodass es um eine Querachse (64) senkrecht zur Visierachse des Endoskops kippen kann,
der Träger (54) an das distale Ende der Bedienungshülse (27) gekuppelt ist, sodass eine Längsverschiebung der besagten Bedienungshülse ein Kippen des Prisma-Trägers (54) um die Querachse (64) hervorruft.

9. Dreh-Endoskop mit abgelenkter Fernvisur gemäß einem beliebigen der vorausgehenden Ansprüche, **dadurch gekennzeichnet, dass**:
das distale Ablenkprisma (3) ein Prisma mit partieller Reflexion ist, das eine unidirektionale Inversion des vom besagten Prisma übertragenen Bildes durchführt,
ein Korrekturprisma (7) mit partieller Reflexion, das eine unidirektionale Inversion des vom besagten Korrekturprisma übertragenen Bildes vornimmt, untrennbar im proximalen Ende der drehenden Hülse (18) untergebracht und radial so positioniert ist, dass das vom besagten Korrekturprisma (5) übertragene Bild keine Inversion bezüglich der Wirklichkeit aufweist.

10. Dreh-Endoskop mit abgelenkter Fernvisur gemäß einem beliebigen der Ansprüche 1 bis 8, **dadurch gekennzeichnet, dass**:
das distale Ablenkprisma (3) ein Prisma mit partieller Reflexion ist, das durch eine unidirektionale Inversion des vom besagten Prisma übertragenen Bildes gekennzeichnet ist,
ein Korrekturprisma mit partieller Reflexion, das durch eine unidirektionale Inversion des vom besagten Korrekturprisma übertragenen Bildes gekennzeichnet ist, untrennbar zwischen zwei Linsen (5) in der inneren Hülse (29) untergebracht und radial so positioniert ist, dass das vom der Okularlinse (6) übertragene Bild keine Inversion bezüglich der Wirklichkeit aufweist.

11. Dreh-Endoskop mit abgelenkter Fernvisur gemäß einem beliebigen der Ansprüche 1 bis 8, **dadurch gekennzeichnet, dass**:
das distale Ablenkprisma (3) ein Prisma mit totaler Reflexion ist, das durch eine bidirektionale Inversion des vom besagten Prisma übertragenen Bildes gekennzeichnet ist,
die das optische Bildtransportsystem bildende "Linsenstraße" (5) so berechnet ist, dass das von der Okularlinse (6) übermittelte Bild keine Inversion bezüglich der Wirklichkeit aufweist.

12. Dreh-Endoskop mit abgelenkter Fernvisur gemäß einem beliebigen der vorausgehenden Ansprüche, **dadurch gekennzeichnet, dass** es eine Anschlagvorrichtung besitzt, die dazu dient, den Drehbereich des die Drehung der Visierachse steuernden Rings (30) zu begrenzen.

13. Dreh-Endoskop mit abgelenkter Fernvisur gemäß Anspruch 12, **dadurch gekennzeichnet, dass** die Anschlagvorrichtung einen Längsstift (31) besitzt, der mit der proximalen Seite des die Drehung der Visierachse steuernden Rings (30) untrennbar verbunden ist, wobei der besagte Stift in das Innere des zylindrischen Griffs (9) ragt, sodass er in Kontakt mit einem inneren, mit dem besagten Griff untrennbar verbundenen radialen Stift (32) kommen kann.

14. Dreh-Endoskop mit abgelenkter Fernvisur gemäß Anspruch 12 oder 13, **dadurch gekennzeichnet, dass** es eine Beleuchtungsvorrichtung besitzt, die aus einem Bündel (25) optischer Fasern besteht, das im Rundvolumen zwischen der inneren und äußeren Hülse (28, 29) untergebracht ist, wobei das distale Ende des besagten Bündels in mindestens ein Beleuchtungsfenster einmündet, das im distalen Teil der äußeren Hülse (28) so angelegt ist, dass das Beleuchtungsfeld das Sichtfeld der Sonde überdeckte, und der proximale Teil des Faserbündels (25) in das Innere des Griffs (9) durch eine im distalen Teil der drehenden Hülse (18) angelegten Öffnung (34) mündet.

15. Dreh-Endoskop mit abgelenkter Fernvisur gemäß Anspruch 12, **dadurch gekennzeichnet, dass** der proximale Teil des Faserbündels (25) kreisförmig um die drehende Hülse (18) gelegt ist, um zu verhindern, dass die Drehung der Sonde Belastungen zur Folge hat, welche die Fasern des besagten Bündels brechen können.
